# EUROPEAN PATENT SPECIFICATION

(11) **EP 2 274 439 B1**
(45) Date of publication and mention of the grant of the patent: **01.10.2014**
(21) Application number: 09729690.9
(22) Date of filing: 10.04.2009
(51) Int. Cl.: G01N 33/574

(54) **METHOD FOR PROFILING DRUG COMPOUNDS**
VERFAHREN ZUR ERSTELLUNG VON PROFILEN FÜR ARZNEISTOFFVERBINDUNGEN
MÉTHODE PERMETTANT L ÉTABLISSEMENT DE PROFILS DE COMPOSÉS MÉDICAMENTEUX

(30) Priority: 11.04.2008 EP 08154419
(43) Date of publication of application: 19.01.2011
(73) Proprietor: PamGene B.V., 5211 's Hertogenbosch (NL)
(72) Inventor: HOUTMAN, René, NL-4105VG Culemborg (NL); RUIJTENBEEK, Robby, NL-3524BK Utrecht (NL)
(74) Representative: De Clercq, Ann G. Y.
(86) International application number: PCT/EP2009/054358
(87) International publication number: WO 2009/125020

(56) References cited:
- WO-A2-2008/049930
- TUYNMAN JURRIAAN B ET AL: "Cyclooxygenase-2 inhibition inhibits c-Met kinase activity and Wnt activity in colon cancer." CANCER RESEARCH 15 FEB 2008, vol. 68, no. 4, 15 February 2008 (2008-02-15), pages 1213-1220, XP002487453 ISSN: 1538-7445
- VAN BEUNINGEN RINIC ET AL: "USE OF FLOW-THROUGH PEPTIDE-MICROARRAYS FOR CEN-LYSATE PROFILING" PROCEEDINGS OF THE ANNUAL MEETING OF THE AMERICAN ASSOCIATIONFOR CANCER RESEARCH, NEW YORK, NY, vol. 47, 1 April 2006 (2006-04-01), pages 1088-1089, XP001248490 ISSN: 0197-016X
- LEMEER SIMONE ET AL: "Protein-tyrosine kinase activity profiling in knock down zebrafish embryos" PLOS ONE, PUBLIC LIBRARY OF SCIENCE, SAN FRANCISCO, CA, US, vol. 2, no. 7, 1 January 2007 (2007-01-01), page e581, XP002469011 ISSN: 1932-6203
- DIKS SANDER H ET AL: "Kinome profiling for studying lipopolysaccharide signal transduction in human peripheral blood mononuclear cells" JOURNAL OF BIOLOGICAL CHEMISTRY, vol. 279, no. 47, 19 November 2004 (2004-11-19), pages 49206-49213, XP002487578 ISSN: 0021-9258
- KOK KLAARTJE ET AL: "Kinome profiling for studying signal transduction" GASTROENTEROLOGY, vol. 126, no. 4, Suppl. 2, April 2004 (2004-04), pages A414-A415, XP002487579 & DIGESTIVE DISEASE WEEK/105TH ANNUAL MEETING OF THE AMERICAN-GASTROENTEROLOGICAL-ASSOCIATION; NEW ORLEANS, LA, USA; MAY 16 20, 2004 ISSN: 0016-5085
- MORI ET AL: "Evaluation of protein kinase activities of cell lysates using peptide microarrays based on surface plasmon resonance imaging", ANALYTICAL BIOCHEMISTRY, ACADEMIC PRESS INC, NEW YORK, vol. 375, no. 2, 15 December 2007 (2007-12-15), pages 223-231, XP022519957, ISSN: 0003-2697, DOI: 10.1016/J.AB.2007.12.011
- SHAWN B. BRUEGGEMEIER ET AL: 'Protein-Acrylamide Copolymer Hydrogels for Array-Based Detection of Tyrosine Kinase Activity from Cell Lysates' BIOMACROMOLECULES vol. 6, no. 5, 01 September 2005, pages 2765 - 2775, XP055017460 DOI: 10.1021/bm050257v ISSN: 1525-7797

### SEQUENCE LISTING

<110> PamGene B.V.
<120> Method for profiling drug compounds
<130> PAM-051-PCT
<150> EP 08154419.9
   <151> 2008-04-11
<160> 157
<170> PatentIn version 3.3
<210> 1
   <211> 13
   <212> PRT
   <213> Artificial
<220>
   <223> peptide kinase substrate
<400> 1
   Leu Asp Gly Glu Asn Ile Tyr Ile Arg His Ser Asn Leu
   1 5 10
<210> 2
   <211> 13
   <212> PRT
   <213> Artificial
<220>
   <223> peptide kinase substrate
<400> 2
   Arg Leu Asp Gly Glu Asn Ile Tyr Ile Arg His Ser Asn
   1 5 10
<210> 3
   <211> 13
   <212> PRT
   <213> Artificial
<220>
   <223> peptide kinase substrate
<400> 3
   Trp Gly Arg Gly Thr Asp Glu Tyr Phe Ile Arg Lys Pro
   1 5 10
<210> 4
   <211> 13
   <212> PRT
   <213> Artificial
<220>
   <223> peptide kinase substrate
<400> 4
   Tyr Ile Ser Lys Ala Glu Glu Tyr Phe Leu Leu Lys Ser
   1 5 10
<210> 5
   <211> 13
   <212> PRT
   <213> Artificial
<220>
   <223> peptide kinase substrate
<400> 5
   Glu Arg Glu Gly Ser Lys Arg Tyr Cys Ile Gln Thr Lys
   1               5                   10
<210> 6
   <211> 13
   <212> PRT
   <213> Artificial
<220>
   <223> peptide kinase substrate
<400> 6
   Ile Glu Asn Glu Glu Gln Glu Tyr Val Gln Thr Val Lys
   1               5                   10
<210> 7
   <211> 13
   <212> PRT
   <213> Artificial
<220>
   <223> peptide kinase substrate
<400> 7
   His Ser Thr Pro Pro Ser Ala Tyr Gly Ser Val Lys Ala
   1               5                   10
<210> 8
   <211> 12
   <212> PRT
   <213> Artificial
<220>
   <223> peptide kinase substrate
<400> 8
   Glu Ala Ile Tyr Ala Ala Pro Phe Ala Lys Lys Lys
   1               5                   10
<210> 9
   <211> 13
   <212> PRT
   <213> Artificial
<220>
   <223> peptide kinase substrate
<400> 9
   Thr Glu Ala Thr Ala Thr Asp Tyr His Thr Thr Ser His
   1 5 10
<210> 10
   <211> 13
   <212> PRT
   <213> Artificial
<220>
   <223> peptide kinase substrate
<400> 10
   Thr Glu Ala Ser Gly Tyr Ile Ser Ser Leu Glu Tyr Pro
   1 5 10
<210> 11
   <211> 13
   <212> PRT
   <213> Artificial
<220>
   <223> peptide kinase substrate
<400> 11
   Phe Asp Lys Asp Gly Asn Gly Tyr Ile Ser Ala Ala Glu
   1 5 10
<210> 12
   <211> 13
   <212> PRT
   <213> Artificial
<220>
   <223> peptide kinase substrate
<400> 12
   Lys Asp Gly Asn Gly Tyr Ile Ser Ala Ala Glu Leu Arg
   1 5 10
<210> 13
   <211> 13
   <212> PRT
   <213> Artificial
<220>
   <223> peptide kinase substrate
<400> 13
   Glu Gly Glu Glu Asp Thr Glu Tyr Met Thr Pro Ser Ser
   1 5 10
<210> 14
   <211> 13
   <212> PRT
   <213> Artificial
<220>
   <223> peptide kinase substrate
<400> 14
   Lys Asp Lys Met Ala Glu Ala Tyr Ser Glu Ile Gly Met
   1 5 10
<210> 15
   <211> 13
   <212> PRT
   <213> Artificial
<220>
   <223> peptide kinase substrate
<400> 15
   Ser Thr Ala Thr Lys Asp Thr Tyr Asp Ala Leu His Met
   1 5 10
<210> 16
   <211> 13
   <212> PRT
   <213> Artificial
<220>
   <223> peptide kinase substrate
<400> 16
   Glu Tyr Glu Asp Glu Asn Leu Tyr Glu Gly Leu Asn Leu
   1 5 10
<210> 17
   <211> 13
   <212> PRT
   <213> Artificial
<220>
   <223> peptide kinase substrate
<400> 17
   Glu Lys Ile Gly Glu Gly Thr Tyr Gly Val Val Tyr Lys
   1 5 10
<210> 18
   <211> 13
   <212> PRT
   <213> Artificial
<220>
   <223> peptide kinase substrate
<400> 18
   Gly Leu Ala Lys Ser Phe Gly Ser Pro Asn Arg Ala Tyr
   1 5 10
<210> 19
   <211> 13
   <212> PRT
   <213> Artificial
<220>
   <223> peptide kinase substrate
<400> 19
   Gln Lys Arg Arg Glu Ile Leu Ser Arg Arg Pro Ser Tyr
   1 5 10
<210> 20
   <211> 13
   <212> PRT
   <213> Artificial
<220>
   <223> peptide kinase substrate
<400> 20
   Gly Pro Pro Glu Pro Gly Pro Tyr Ala Gln Pro Ser Val
   1 5 10
<210> 21
   <211> 13
   <212> PRT
   <213> Artificial
<220>
   <223> peptide kinase substrate
<400> 21
   Val Ala Asp Ile Asp Gly Gln Tyr Ala Met Thr Arg Ala
   1 5 10
<210> 22
   <211> 13
   <212> PRT
   <213> Artificial
<220>
   <223> peptide kinase substrate
<400> 22
   Gly Ile Val Tyr Ala Val Ser Ser Asp Arg Phe Arg Ser
   1 5 10
<210> 23
   <211> 13
   <212> PRT
   <213> Artificial
<220>
   <223> peptide kinase substrate
<400> 23
   Leu Leu Leu Ser Asn Pro Ala Tyr Arg Leu Leu Leu Ala
   1 5 10
<210> 24
   <211> 13
   <212> PRT
   <213> Artificial
<220>
   <223> peptide kinase substrate
<400> 24
   Phe Gly Met Ser Arg Asn Leu Tyr Ala Gly Asp Tyr Tyr
   1 5 10
<210> 25
   <211> 13
   <212> PRT
   <213> Artificial
<220>
   <223> peptide kinase substrate
<400> 25
   Arg Asn Leu Tyr Ser Gly Asp Tyr Tyr Arg Ile Gln Gly
   1 5 10
<210> 26
   <211> 13
   <212> PRT
   <213> Artificial
<220>
   <223> peptide kinase substrate
<400> 26
   Lys His Asp Thr Glu Met Lys Tyr Tyr Ile Val His Leu
   1 5 10
<210> 27
   <211> 13
   <212> PRT
   <213> Artificial
<220>
   <223> peptide kinase substrate
<400> 27
   Cys Gln Leu Gly Gln Arg Ile Tyr Gln Tyr Ile Gln Ser
   1 5 10
<210> 28
   <211> 13
   <212> PRT
   <213> Artificial
<220>
   <223> peptide kinase substrate
<400> 28
   Gly Gly Thr Asp Glu Gly Ile Tyr Asp Val Pro Leu Leu
   1 5 10
<210> 29
   <211> 13
   <212> PRT
   <213> Artificial
<220>
   <223> peptide kinase substrate
<400> 29
   Gly Gly Thr Asp Glu Gly Ile Phe Asp Val Pro Leu Leu
   1 5 10
<210> 30
   <211> 13
   <212> PRT
   <213> Artificial
<220>
   <223> peptide kinase substrate
<400> 30
   Glu Asp Ser Phe Leu Gln Arg Tyr Ser Ser Asp Pro Thr
   1 5 10
<210> 31
   <211> 13
   <212> PRT
   <213> Artificial
<220>
   <223> peptide kinase substrate
<400> 31
   Gly Ser Val Gln Asn Pro Val Tyr His Asn Gln Pro Leu
   1 5 10
<210> 32
   <211> 13
   <212> PRT
   <213> Artificial
<220>
   <223> peptide kinase substrate
<400> 32
   Ala Pro Ser Arg Asp Pro His Tyr Gln Asp Pro His Ser
   1 5 10
<210> 33
   <211> 13
   <212> PRT
   <213> Artificial
<220>
   <223> peptide kinase substrate
<400> 33
   Ile Ser Leu Asp Asn Pro Asp Tyr Gln Gln Asp Phe Phe
   1 5 10
<210> 34
   <211> 13
   <212> PRT
   <213> Artificial
<220>
   <223> peptide kinase substrate
<400> 34
   Ser Thr Ala Glu Asn Ala Glu Tyr Leu Arg Val Ala Pro
   1 5 10
<210> 35
   <211> 13
   <212> PRT
   <213> Artificial
<220>
   <223> peptide kinase substrate
<400> 35
   Leu Gly Ala Glu Glu Lys Glu Tyr His Ala Glu Gly Gly
   1 5 10
<210> 36
   <211> 13
   <212> PRT
   <213> Artificial
<220>
   <223> peptide kinase substrate
<400> 36
   Met Thr Phe Gly Ser Lys Pro Tyr Asp Gly Ile Pro Ala
   1 5 10
<210> 37
   <211> 13
   <212> PRT
   <213> Artificial
<220>
   <223> peptide kinase substrate
<400> 37
   Ser Gly Ala Ser Thr Gly Ile Tyr Glu Ala Leu Glu Leu
   1 5 10
<210> 38
   <211> 13
   <212> PRT
   <213> Artificial
<220>
   <223> peptide kinase substrate
<400> 38
   Leu Asp Asp Phe Asp Gly Thr Tyr Glu Thr Gln Gly Gly
   1 5 10
<210> 39
   <211> 13
   <212> PRT
   <213> Artificial
<220>
   <223> peptide kinase substrate
<400> 39
   Gln Leu Lys Pro Leu Lys Thr Tyr Val Asp Pro His Thr
   1 5 10
<210> 40
   <211> 13
   <212> PRT
   <213> Artificial
<220>
   <223> peptide kinase substrate
<400> 40
   Glu Asp Asp Pro Glu Ala Thr Tyr Thr Thr Ser Gly Gly
   1 5 10
<210> 41
   <211> 13
   <212> PRT
   <213> Artificial
<220>
   <223> peptide kinase substrate
<400> 41
   Leu Asn Gln Gly Val Arg Thr Tyr Val Asp Pro Phe Thr
   1               5                   10
<210> 42
   <211> 13
   <212> PRT
   <213> Artificial
<220>
   <223> peptide kinase substrate
<400> 42
   Gln Ala Ile Lys Met Asp Arg Tyr Lys Asp Asn Phe Thr
   1               5                   10
<210> 43
   <211> 13
   <212> PRT
   <213> Artificial
<220>
   <223> peptide kinase substrate
<400> 43
   Thr Tyr Ile Asp Pro Glu Thr Tyr Glu Asp Pro Asn Arg
   1               5                   10
<210> 44
   <211> 13
   <212> PRT
   <213> Artificial
<220>
   <223> peptide kinase substrate
<400> 44
   Asp Asp Thr Ser Asp Pro Thr Tyr Thr Ser Ser Leu Gly
   1               5                   10
<210> 45
   <211> 13
   <212> PRT
   <213> Artificial
<220>
   <223> peptide kinase substrate
<400> 45
   Ser Ala Ile Lys Met Val Gln Tyr Arg Asp Ser Phe Leu
   1 5 10
<210> 46
   <211> 13
   <212> PRT
   <213> Artificial
<220>
   <223> peptide kinase substrate
<400> 46
   Ile Gly His Gly Thr Lys Val Tyr Ile Asp Pro Phe Thr
   1 5 10
<210> 47
   <211> 13
   <212> PRT
   <213> Artificial
<220>
   <223> peptide kinase substrate
<400> 47
   Ser Glu His Ala Gln Asp Thr Tyr Leu Val Leu Asp Lys
   1 5 10
<210> 48
   <211> 13
   <212> PRT
   <213> Artificial
<220>
   <223> peptide kinase substrate
<400> 48
   Ala Ser Ala Ala Ser Phe Glu Tyr Thr Ile Leu Asp Pro
   1 5 10
<210> 49
   <211> 13
   <212> PRT
   <213> Artificial
<220>
   <223> peptide kinase substrate
<400> 49
   Pro Thr Ala Glu Asn Pro Glu Tyr Leu Gly Leu Asp Val
   1 5 10
<210> 50
   <211> 13
   <212> PRT
   <213> Artificial
<220>
   <223> peptide kinase substrate
<400> 50
   Leu Asp Ile Asp Glu Thr Glu Tyr His Ala Asp Gly Gly
   1 5 10
<210> 51
   <211> 13
   <212> PRT
   <213> Artificial
<220>
   <223> peptide kinase substrate
<400> 51
   Pro Ile Ser Thr Ile Asp Val Tyr Met Ile Met Val Lys
   1 5 10
<210> 52
   <211> 13
   <212> PRT
   <213> Artificial
<220>
   <223> peptide kinase substrate
<400> 52
   Gln Ala Leu Asp Asn Pro Glu Tyr His Asn Ala Ser Asn
   1 5 10
<210> 53
   <211> 13
   <212> PRT
   <213> Artificial
<220>
   <223> peptide kinase substrate
<400> 53
   Ile Val Ala Glu Asn Pro Glu Tyr Leu Ser Glu Phe Ser
   1               5                   10
<210> 54
   <211> 13
   <212> PRT
   <213> Artificial
<220>
   <223> peptide kinase substrate
<400> 54
   Arg Leu Gln Arg Arg Arg Gly Ser Ser Ile Pro Gln Phe
   1               5                   10
<210> 55
   <211> 13
   <212> PRT
   <213> Artificial
<220>
   <223> peptide kinase substrate
<400> 55
   Asp Ser Lys Asn Phe Asp Asp Tyr Met Lys Ser Leu Gly
   1               5                   10
<210> 56
   <211> 13
   <212> PRT
   <213> Artificial
<220>
   <223> peptide kinase substrate
<400> 56
   Arg Tyr Met Glu Asp Ser Thr Tyr Tyr Lys Ala Ser Lys
   1               5                   10
<210> 57
   <211> 13
   <212> PRT
   <213> Artificial
<220>
   <223> peptide kinase substrate
<400> 57
   Arg Tyr Ile Glu Asp Glu Asp Tyr Tyr Lys Ala Ser Val
   1 5 10
<210> 58
   <211> 13
   <212> PRT
   <213> Artificial
<220>
   <223> peptide kinase substrate
<400> 58
   Arg Gln Glu Asp Gly Gly Val Tyr Ser Ser Ser Gly Leu
   1 5 10
<210> 59
   <211> 13
   <212> PRT
   <213> Artificial
<220>
   <223> peptide kinase substrate
<400> 59
   Arg Glu Glu Ala Asp Gly Val Tyr Ala Ala Ser Gly Gly
   1 5 10
<210> 60
   <211> 13
   <212> PRT
   <213> Artificial
<220>
   <223> peptide kinase substrate
<400> 60
   Ala Leu Thr Ser Asn Gln Glu Tyr Leu Asp Leu Ser Met
   1 5 10
<210> 61
   <211> 13
   <212> PRT
   <213> Artificial
<220>
   <223> peptide kinase substrate
<400> 61
   Thr Leu Thr Thr Asn Glu Glu Tyr Leu Asp Leu Ser Gln
   1 5 10
<210> 62
   <211> 13
   <212> PRT
   <213> Artificial
<220>
   <223> peptide kinase substrate
<400> 62
   Asp Val His Asn Leu Asp Tyr Tyr Lys Lys Thr Thr Asn
   1 5 10
<210> 63
   <211> 13
   <212> PRT
   <213> Artificial
<220>
   <223> peptide kinase substrate
<400> 63
   Thr Val Thr Ser Thr Asp Glu Tyr Leu Asp Leu Ser Ala
   1 5 10
<210> 64
   <211> 13
   <212> PRT
   <213> Artificial
<220>
   <223> peptide kinase substrate
<400> 64
   Lys Val Asp Asn Glu Asp Ile Tyr Glu Ser Arg His Glu
   1 5 10
<210> 65
   <211> 13
   <212> PRT
   <213> Artificial
<220>
   <223> peptide kinase substrate
<400> 65
   Arg Gly Glu Pro Asn Val Ser Tyr Ile Cys Ser Arg Tyr
   1 5 10
<210> 66
   <211> 13
   <212> PRT
   <213> Artificial
<220>
   <223> peptide kinase substrate
<400> 66
   Asp Gly Lys Lys Arg Lys Arg Ser Arg Lys Glu Ser Tyr
   1 5 10
<210> 67
   <211> 13
   <212> PRT
   <213> Artificial
<220>
   <223> peptide kinase substrate
<400> 67
   Ser Leu Gly Phe Lys Arg Ser Tyr Glu Glu His Ile Pro
   1 5 10
<210> 68
   <211> 13
   <212> PRT
   <213> Artificial
<220>
   <223> peptide kinase substrate
<400> 68
   Tyr Ala Ser Ser Asn Pro Glu Tyr Leu Ser Ala Ser Asp
   1 5 10
<210> 69
   <211> 13
   <212> PRT
   <213> Artificial
<220>
   <223> peptide kinase substrate
<400> 69
   Ser Ser Glu Asp Leu Ser Ala Tyr Ala Ser Ile Ser Phe
   1 5 10
<210> 70
   <211> 13
   <212> PRT
   <213> Artificial
<220>
   <223> peptide kinase substrate
<400> 70
   Gly Gly Gly Gly Gly Glu Phe Tyr Gly Tyr Met Thr Met
   1 5 10
<210> 71
   <211> 13
   <212> PRT
   <213> Artificial
<220>
   <223> peptide kinase substrate
<400> 71
   Ala Ile Glu Thr Asp Lys Glu Tyr Tyr Thr Val Lys Asp
   1 5 10
<210> 72
   <211> 13
   <212> PRT
   <213> Artificial
<220>
   <223> peptide kinase substrate
<400> 72
   Gly Ala Gly Phe Gly Ser Arg Ser Leu Tyr Gly Leu Gly
   1 5 10
<210> 73
   <211> 13
   <212> PRT
   <213> Artificial
<220>
   <223> peptide kinase substrate
<400> 73
   Ser Ala Tyr Gly Gly Leu Thr Ser Pro Gly Leu Ser Tyr
   1 5 10
<210> 74
   <211> 13
   <212> PRT
   <213> Artificial
<220>
   <223> peptide kinase substrate
<400> 74
   Ala Leu Arg Ala Asp Glu Asn Tyr Tyr Lys Ala Gln Thr
   1 5 10
<210> 75
   <211> 13
   <212> PRT
   <213> Artificial
<220>
   <223> peptide kinase substrate
<400> 75
   Met Glu Ser Ile Asp Asp Tyr Val Asn Val Pro Glu Ser
   1 5 10
<210> 76
   <211> 13
   <212> PRT
   <213> Artificial
<220>
   <223> peptide kinase substrate
<400> 76
   Glu Glu Gly Ala Pro Asp Tyr Glu Asn Leu Gln Glu Leu
   1 5 10
<210> 77
   <211> 13
   <212> PRT
   <213> Artificial
<220>
   <223> peptide kinase substrate
<400> 77
   Arg Leu Ile Glu Asp Asn Glu Tyr Thr Ala Arg Glu Gly
   1 5 10
<210> 78
   <211> 13
   <212> PRT
   <213> Artificial
<220>
   <223> peptide kinase substrate
<400> 78
   Arg Asp Ile Tyr Arg Ala Ser Tyr Tyr Arg Arg Gly Asp
   1 5 10
<210> 79
   <211> 13
   <212> PRT
   <213> Artificial
<220>
   <223> peptide kinase substrate
<400> 79
   Ala Arg Thr Ala His Tyr Gly Ser Leu Pro Gln Lys Ser
   1 5 10
<210> 80
   <211> 13
   <212> PRT
   <213> Artificial
<220>
   <223> peptide kinase substrate
<400> 80
   Phe Gly Tyr Gly Gly Arg Ala Ser Asp Tyr Lys Ser Ala
   1 5 10
<210> 81
   <211> 13
   <212> PRT
   <213> Artificial
<220>
   <223> peptide kinase substrate
<400> 81
   Gly Arg Ala Ser Asp Tyr Lys Ser Ala His Lys Gly Phe
   1 5 10
<210> 82
   <211> 13
   <212> PRT
   <213> Artificial
<220>
   <223> peptide kinase substrate
<400> 82
   Arg Asp Met Tyr Asp Lys Glu Tyr Tyr Ser Val His Asn
   1 5 10
<210> 83
   <211> 13
   <212> PRT
   <213> Artificial
<220>
   <223> peptide kinase substrate
<400> 83
   His Thr Gly Phe Leu Thr Glu Tyr Val Ala Thr Arg Trp
   1 5 10
<210> 84
   <211> 13
   <212> PRT
   <213> Artificial
<220>
   <223> peptide kinase substrate
<400> 84
   Ile Met Leu Asn Ser Lys Gly Tyr Thr Lys Ser Ile Asp
   1 5 10
<210> 85
   <211> 13
   <212> PRT
   <213> Artificial
<220>
   <223> peptide kinase substrate
<400> 85
   Ala Glu His Gln Tyr Phe Met Thr Glu Tyr Val Ala Thr
   1 5 10
<210> 86
   <211> 13
   <212> PRT
   <213> Artificial
<220>
   <223> peptide kinase substrate
<400> 86
   Thr Ser Phe Met Met Thr Pro Tyr Val Val Thr Arg Tyr
   1 5 10
<210> 87
   <211> 13
   <212> PRT
   <213> Artificial
<220>
   <223> peptide kinase substrate
<400> 87
   Ala Asp Ser Glu Met Thr Gly Tyr Val Val Thr Arg Trp
   1 5 10
<210> 88
   <211> 13
   <212> PRT
   <213> Artificial
<220>
   <223> peptide kinase substrate
<400> 88
   Arg His Thr Asp Asp Glu Met Thr Gly Tyr Val Ala Thr
   1 5 10
<210> 89
   <211> 13
   <212> PRT
   <213> Artificial
<220>
   <223> peptide kinase substrate
<400> 89
   Gln Arg Ser Arg Lys Arg Leu Ser Gln Asp Ala Tyr Arg
   1 5 10
<210> 90
   <211> 13
   <212> PRT
   <213> Artificial
<220>
   <223> peptide kinase substrate
<400> 90
   Ala Lys Ala Leu Gly Lys Arg Thr Ala Lys Tyr Arg Trp
   1 5 10
<210> 91
   <211> 13
   <212> PRT
   <213> Artificial
<220>
   <223> peptide kinase substrate
<400> 91
   His Ile Ile Glu Asn Pro Gln Tyr Phe Ser Asp Ala Cys
   1 5 10
<210> 92
   <211> 13
   <212> PRT
   <213> Artificial
<220>
   <223> peptide kinase substrate
<400> 92
   Pro Val Ile Glu Asn Pro Gln Tyr Phe Gly Ile Thr Asn
   1 5 10
<210> 93
   <211> 13
   <212> PRT
   <213> Artificial
<220>
   <223> peptide kinase substrate
<400> 93
   Phe Gly Met Ser Arg Asp Val Tyr Ser Thr Asp Tyr Tyr
   1 5 10
<210> 94
   <211> 13
   <212> PRT
   <213> Artificial
<220>
   <223> peptide kinase substrate
<400> 94
   Arg Asp Val Tyr Ser Thr Asp Tyr Tyr Arg Val Gly Gly
   1 5 10
<210> 95
   <211> 13
   <212> PRT
   <213> Artificial
<220>
   <223> peptide kinase substrate
<400> 95
   Asp Asp Ser Ser Ala Tyr Arg Ser Val Asp Glu Val Asn
   1 5 10
<210> 96
   <211> 13
   <212> PRT
   <213> Artificial
<220>
   <223> peptide kinase substrate
<400> 96
   Ser Met Ser Asp Pro Gly Val Ser Tyr Arg Thr Arg Glu
   1 5 10
<210> 97
   <211> 13
   <212> PRT
   <213> Artificial
<220>
   <223> peptide kinase substrate
<400> 97
   Glu Pro His Val Thr Arg Arg Thr Pro Asp Tyr Phe Leu
   1 5 10
<210> 98
   <211> 13
   <212> PRT
   <213> Artificial
<220>
   <223> peptide kinase substrate
<400> 98
   Asn Glu Asn Thr Glu Asp Gln Tyr Ser Leu Val Glu Asp
   1 5 10
<210> 99
   <211> 13
   <212> PRT
   <213> Artificial
<220>
   <223> peptide kinase substrate
<400> 99
   Val Gly Glu Glu Glu His Val Tyr Ser Phe Pro Asn Lys
   1 5 10
<210> 100
   <211> 13
   <212> PRT
   <213> Artificial
<220>
   <223> peptide kinase substrate
<400> 100
   Phe Leu Ser Glu Glu Thr Pro Tyr Ser Tyr Pro Thr Gly
   1 5 10
<210> 101
   <211> 13
   <212> PRT
   <213> Artificial
<220>
   <223> peptide kinase substrate
<400> 101
   Ala Arg Thr Thr Ser Gln Leu Tyr Asp Ala Val Pro Ile
   1 5 10
<210> 102
   <211> 13
   <212> PRT
   <213> Artificial
<220>
   <223> peptide kinase substrate
<400> 102
   Asp Glu Asp Cys Tyr Gly Asn Tyr Asp Asn Leu Leu Ser
   1 5 10
<210> 103
   <211> 13
   <212> PRT
   <213> Artificial
<220>
   <223> peptide kinase substrate
<400> 103
   Lys Lys Asp Thr Glu Thr Val Tyr Ser Glu Val Arg Lys
   1 5 10
<210> 104
   <211> 13
   <212> PRT
   <213> Artificial
<220>
   <223> peptide kinase substrate
<400> 104
   Gln Arg Ser Glu Leu Asp Lys Ser Ser Ala His Ser Tyr
   1 5 10
<210> 105
   <211> 13
   <212> PRT
   <213> Artificial
<220>
   <223> peptide kinase substrate
<400> 105
   Leu Asp Thr Ser Ser Val Leu Tyr Thr Ala Val Gln Pro
   1               5                   10
<210> 106
   <211> 13
   <212> PRT
   <213> Artificial
<220>
   <223> peptide kinase substrate
<400> 106
   Val Ser Ser Asp Gly His Glu Tyr Ile Tyr Val Asp Pro
   1               5                   10
<210> 107
   <211> 13
   <212> PRT
   <213> Artificial
<220>
   <223> peptide kinase substrate
<400> 107
   Arg Pro Pro Ser Ala Glu Leu Tyr Ser Asn Ala Leu Pro
   1               5                   10
<210> 108
   <211> 13
   <212> PRT
   <213> Artificial
<220>
   <223> peptide kinase substrate
<400> 108
   Ser Ser Asn Tyr Met Ala Pro Tyr Asp Asn Tyr Val Pro
   1               5                   10
<210> 109
   <211> 13
   <212> PRT
   <213> Artificial
<220>
   <223> peptide kinase substrate
<400> 109
   Tyr Met Ala Pro Tyr Asp Asn Tyr Val Pro Ser Ala Pro
   1               5                   10
<210> 110
   <211> 13
   <212> PRT
   <213> Artificial
<220>
   <223> peptide kinase substrate
<400> 110
   Glu Gly Ser Phe Glu Ser Arg Tyr Gln Gln Pro Phe Glu
   1               5                   10
<210> 111
   <211> 13
   <212> PRT
   <213> Artificial
<220>
   <223> peptide kinase substrate
<400> 111
   Ile Gly Thr Ala Glu Pro Asp Tyr Gly Ala Leu Tyr Glu
   1               5                   10
<210> 112
   <211> 13
   <212> PRT
   <213> Artificial
<220>
   <223> peptide kinase substrate
<400> 112
   Glu Gly Arg Asn Pro Gly Phe Tyr Val Glu Ala Asn Pro
   1               5                   10
<210> 113
   <211> 13
   <212> PRT
   <213> Artificial
<220>
   <223> peptide kinase substrate
<400> 113
   Leu Arg Pro Asp Ser Glu Ala Ser Gln Ser Pro Gln Tyr
   1 5 10
<210> 114
   <211> 13
   <212> PRT
   <213> Artificial
<220>
   <223> peptide kinase substrate
<400> 114
   Glu Gln Arg Met Lys Glu Ser Ser Phe Tyr Ser Leu Cys
   1 5 10
<210> 115
   <211> 13
   <212> PRT
   <213> Artificial
<220>
   <223> peptide kinase substrate
<400> 115
   Trp Thr Ala Ser Ser Pro Tyr Ser Thr Val Pro Pro Tyr
   1 5 10
<210> 116
   <211> 13
   <212> PRT
   <213> Artificial
<220>
   <223> peptide kinase substrate
<400> 116
   Ser Lys Arg Lys Gly His Glu Tyr Thr Asn Ile Lys Tyr
   1 5 10
<210> 117
   <211> 13
   <212> PRT
   <213> Artificial
<220>
   <223> peptide kinase substrate
<400> 117
   Arg Pro Arg Gly Gln Arg Asp Ser Ser Tyr Tyr Trp Glu
   1 5 10
<210> 118
   <211> 13
   <212> PRT
   <213> Artificial
<220>
   <223> peptide kinase substrate
<400> 118
   Thr Val Asp Gly Lys Glu Ile Tyr Asn Thr Ile Arg Arg
   1 5 10
<210> 119
   <211> 13
   <212> PRT
   <213> Artificial
<220>
   <223> peptide kinase substrate
<400> 119
   Ile Tyr Ile Ser Pro Leu Lys Ser Pro Tyr Lys Ile Ser
   1 5 10
<210> 120
   <211> 13
   <212> PRT
   <213> Artificial
<220>
   <223> peptide kinase substrate
<400> 120
   Val Pro Thr Val Ser Lys Gly Thr Val Glu Gly Asn Tyr
   1 5 10
<210> 121
   <211> 13
   <212> PRT
   <213> Artificial
<220>
   <223> peptide kinase substrate
<400> 121
   Thr Pro Ser Asp Ser Leu Ile Tyr Asp Asp Gly Leu Ser
   1 5 10
<210> 122
   <211> 13
   <212> PRT
   <213> Artificial
<220>
   <223> peptide kinase substrate
<400> 122
   Ala Gln Ala Phe Pro Val Ser Tyr Ser Ser Ser Gly Ala
   1 5 10
<210> 123
   <211> 13
   <212> PRT
   <213> Artificial
<220>
   <223> peptide kinase substrate
<400> 123
   Ser Ala Leu Leu Gly Asp His Tyr Val Gln Leu Pro Ala
   1 5 10
<210> 124
   <211> 13
   <212> PRT
   <213> Artificial
<220>
   <223> peptide kinase substrate
<400> 124
   Tyr Val Gln Leu Pro Ala Thr Tyr Met Asn Leu Gly Pro
   1 5 10
<210> 125
   <211> 13
   <212> PRT
   <213> Artificial
<220>
   <223> peptide kinase substrate
<400> 125
   Val Ser Gln Arg Glu Ala Glu Tyr Glu Pro Glu Thr Val
   1 5 10
<210> 126
   <211> 13
   <212> PRT
   <213> Artificial
<220>
   <223> peptide kinase substrate
<400> 126
   Glu Tyr Glu Pro Glu Thr Val Tyr Glu Val Ala Gly Ala
   1 5 10
<210> 127
   <211> 13
   <212> PRT
   <213> Artificial
<220>
   <223> peptide kinase substrate
<400> 127
   Tyr Gln Ala Glu Glu Asn Thr Tyr Asp Glu Tyr Glu Asn
   1 5 10
<210> 128
   <211> 13
   <212> PRT
   <213> Artificial
<220>
   <223> peptide kinase substrate
<400> 128
   Leu Ala Lys Ala Val Asp Gly Tyr Val Lys Pro Gln Ile
   1 5 10
<210> 129
   <211> 13
   <212> PRT
   <213> Artificial
<220>
   <223> peptide kinase substrate
<400> 129
   Asp Gly Pro Lys Gly Thr Gly Tyr Ile Lys Thr Glu Leu
   1 5 10
<210> 130
   <211> 13
   <212> PRT
   <213> Artificial
<220>
   <223> peptide kinase substrate
<400> 130
   Asn Leu Gln Glu Arg Arg Lys Tyr Leu Lys His Arg Leu
   1 5 10
<210> 131
   <211> 13
   <212> PRT
   <213> Artificial
<220>
   <223> peptide kinase substrate
<400> 131
   Asp Pro Gly Ser Ala Ala Pro Tyr Leu Lys Thr Lys Phe
   1 5 10
<210> 132
   <211> 13
   <212> PRT
   <213> Artificial
<220>
   <223> peptide kinase substrate
<400> 132
   Thr Glu Arg Gly Asp Lys Gly Tyr Val Pro Ser Val Phe
   1 5 10
<210> 133
   <211> 13
   <212> PRT
   <213> Artificial
<220>
   <223> peptide kinase substrate
<400> 133
   Pro Ser Asp Leu Leu Pro Met Ser Pro Ser Val Tyr Ala
   1               5                   10
<210> 134
   <211> 13
   <212> PRT
   <213> Artificial
<220>
   <223> peptide kinase substrate
<400> 134
   Met Gly Lys Asp Gly Arg Gly Tyr Val Pro Ala Thr Ile
   1               5                   10
<210> 135
   <211> 13
   <212> PRT
   <213> Artificial
<220>
   <223> peptide kinase substrate
<400> 135
   Asn Tyr Leu Arg Arg Arg Leu Ser Asp Ser Asn Phe Met
   1               5                   10
<210> 136
   <211> 13
   <212> PRT
   <213> Artificial
<220>
   <223> peptide kinase substrate
<400> 136
   Ser Gly Tyr Ser Ser Pro Gly Ser Pro Gly Thr Pro Gly
   1               5                   10
<210> 137
   <211> 13
   <212> PRT
   <213> Artificial
<220>
   <223> peptide kinase substrate
<400> 137
   Arg Tyr Phe Leu Asp Asp Gln Tyr Thr Ser Ser Ser Gly
   1               5                   10
<210> 138
   <211> 13
   <212> PRT
   <213> Artificial
<220>
   <223> peptide kinase substrate
<400> 138
   Ser Asp Thr Glu Glu Gln Glu Tyr Glu Glu Glu Gln Pro
   1 5 10
<210> 139
   <211> 13
   <212> PRT
   <213> Artificial
<220>
   <223> peptide kinase substrate
<400> 139
   Lys Ile Tyr Ser Gly Asp Tyr Tyr Arg Gln Gly Cys Ala
   1 5 10
<210> 140
   <211> 13
   <212> PRT
   <213> Artificial
<220>
   <223> peptide kinase substrate
<400> 140
   Asp Phe Gly Leu Ala Arg Asp Ile Tyr Lys Asn Pro Asp
   1 5 10
<210> 141
   <211> 13
   <212> PRT
   <213> Artificial
<220>
   <223> peptide kinase substrate
<400> 141
   Asp Ile Phe Lys Asn Pro Asp Tyr Val Arg Lys Gly Asp
   1 5 10
<210> 142
   <211> 13
   <212> PRT
   <213> Artificial
<220>
   <223> peptide kinase substrate
<400> 142
   Lys Asn Pro Asp Tyr Val Arg Lys Gly Asp Thr Arg Leu
   1 5 10
<210> 143
   <211> 13
   <212> PRT
   <213> Artificial
<220>
   <223> peptide kinase substrate
<400> 143
   Val Gln Gln Asp Gly Lys Asp Tyr Ile Pro Ile Asn Ala
   1 5 10
<210> 144
   <211> 13
   <212> PRT
   <213> Artificial
<220>
   <223> peptide kinase substrate
<400> 144
   Gly Ser Ser Asp Asp Val Arg Tyr Val Asn Ala Phe Lys
   1 5 10
<210> 145
   <211> 13
   <212> PRT
   <213> Artificial
<220>
   <223> peptide kinase substrate
<400> 145
   Ala Thr Ser Met Phe Asp Asp Tyr Gln Gly Asp Ser Ser
   1 5 10
<210> 146
   <211> 13
   <212> PRT
   <213> Artificial
<220>
   <223> peptide kinase substrate
<400> 146
   Ser Ser Ser Pro Pro Pro Asp Tyr Asn Ser Val Val Leu
   1 5 10
<210> 147
   <211> 13
   <212> PRT
   <213> Artificial
<220>
   <223> peptide kinase substrate
<400> 147
   Asp Tyr Asn Ser Val Val Leu Tyr Ser Thr Pro Pro Ile
   1 5 10
<210> 148
   <211> 13
   <212> PRT
   <213> Artificial
<220>
   <223> peptide kinase substrate
<400> 148
   Asp Phe Gly Leu Ala Arg Asp Ile Tyr Lys Asp Pro Asp
   1 5 10
<210> 149
   <211> 13
   <212> PRT
   <213> Artificial
<220>
   <223> peptide kinase substrate
<400> 149
   Asp Ile Tyr Lys Asp Pro Asp Tyr Val Arg Lys Gly Asp
   1 5 10
<210> 150
   <211> 13
   <212> PRT
   <213> Artificial
<220>
   <223> peptide kinase substrate
<400> 150
   Ala Gln Gln Asp Gly Lys Asp Tyr Ile Val Leu Pro Ile
   1 5 10
<210> 151
   <211> 13
   <212> PRT
   <213> Artificial
<220>
   <223> peptide kinase substrate
<400> 151
   Val Ser Asp Pro Lys Phe His Tyr Asp Asn Thr Ala Gly
   1 5 10
<210> 152
   <211> 13
   <212> PRT
   <213> Artificial
<220>
   <223> peptide kinase substrate
<400> 152
   Arg Phe Arg Gln Gly Lys Asp Tyr Val Gly Ala Ile Pro
   1 5 10
<210> 153
   <211> 13
   <212> PRT
   <213> Artificial
<220>
   <223> peptide kinase substrate
<400> 153
   Glu Glu Ala Pro Glu Asp Leu Tyr Lys Asp Phe Leu Thr
   1 5 10
<210> 154
   <211> 13
   <212> PRT
   <213> Artificial
<220>
   <223> peptide kinase substrate
<400> 154
   Asp Ile Tyr Lys Asp Pro Asp Tyr Val Arg Lys Gly Ser
   1               5                   10
<210> 155
   <211> 13
   <212> PRT
   <213> Artificial
<220>
   <223> peptide kinase substrate
<400> 155
   Lys Ser Phe Leu Asp Ser Gly Tyr Arg Ile Leu Gly Ala
   1               5                   10
<210> 156
   <211> 13
   <212> PRT
   <213> Artificial
<220>
   <223> peptide kinase substrate
<400> 156
   Ala Leu Gly Ala Asp Asp Ser Tyr Tyr Thr Ala Arg Ser
   1               5                   10
<210> 157
   <211> 13
   <212> PRT
   <213> Artificial
<220>
   <223> peptide kinase substrate
<400> 157
   Leu Arg Thr His Asn Gly Ala Ser Pro Tyr Gln Cys Thr
   1               5                   10

## Description

### Field of the invention

The present invention relates to systems and methods for testing kinase inhibition using an array of substrates, in particular protein kinase substrates, immobilized on a porous matrix. More particularly, the method provides kinase inhibition profiles of kinase inhibitors using phosphorylation patterns.

### Background

Signal transduction is one of the most important areas of investigation in biological research, and involves many types of interactions. One of the major mechanisms frequently employed by cells to regulate their activity, and in particular to regulate signal transduction processes, involves changes in protein phosphorylation. Since protein phosphorylation is involved in numerous regulatory events in cells. For example, specific phosphorylation of various proteins, mediated through phosphorylation by kinases or dephosphorylation by phosphatases, often provides a mechanism through which cell surface signalling pathways transmit and integrate information into the nucleus. Protein phosphorylation commonly occurs on the hydroxy group of an amino acid such as tyrosine, serine, or threonine within a polypeptide, and changes in the phosphorylation state of these polypeptides regulate many aspects of cellular metabolism, growth, and/or differentiation.

It is therefore not unexpected that defects in protein kinases and phosphatases or their regulation can be associated with various diseases. It has, for example, already been established that protein kinases, both tyrosine, serine and threonine kinases, play an important role in signalling pathways that are known to play key roles in tumor development and progression. A deregulation of protein kinase activity has been observed in many malignant neoplasms. Unusual protein kinase activity has also been discovered in pediatric brain tumors. However only a limited number of the known protein kinases have been investigated so far.

Due to their association with various diseases, protein kinases and phosphatases are interesting targets for therapeutic intervention, and many clinically useful drugs have been designed or found to act on protein kinases or phosphatases. Kinase inhibitors, such as Imatinib and Gefitinib, inhibit specific kinases and are therefore useful in the treatment of cancer.

Since as many as up to 1000 kinases and 500 phosphatases in the human genome are thought to be involved in phosphorylation processes, kinases and phosphatases encompass a large number of potential new drug targets.

The methods for measuring protein phosphorylation rely on the incorporation and measurement of radioisotopes into target proteins, but since the incorporation is very low, these radioisotope assays are relatively insensitive. Another problem in these experiments is the promiscuity of the kinase for non-physiological substrates. Consequently, radioisotope based assays will often not provide useful data on preferential specificity of an inhibitor against multiple kinases.

Furthermore, other assays where protein phosphorylation is investigated test single kinases. Moreover, such assays require tedious sample processing steps and will therefore present significant obstacles towards reliable systems (e.g., relatively high inter-sample and intra-sample variation due to numerous processing steps). Therefore, there is a need for reliable high-throughput assays that study protein phosphorylation and to allow applications in the identification of biomarkers that can help predict efficacies of kinase inhibitor therapies.

J. Tuynman et al.( Cancer research, Vol. 68, no. 4, pp. 1213-1220) provides an analysis of the effects of the selective cyclooxygenase-2 inhibitor celecoxib on the signal transduction in colorectal cancer cells. For this analysis cells are harvested and lysed after the treatment with celecoxib, and after some process steps the cell lysate is incubated on PepChip arrays containing 1176 peptide kinase substrates. After exposure, the phospohorylation profile is evaluated. S. Lemeer et al. (PLoS ONE, vol. 2, no. 7. pp. e581) provides experiments where peptide chip arrays are used to determine the protein-tyrosine kinase profiles of zebrafish embryos. S. Diks et al. (Journal of Biological Chemistry, Vol. 279, no. 47, pp. 49206-49213) provides experiments with a peptide array, allowing to obtain the phosphorylation profile of peripheral blood mononuclear cells induced by lipopolysaccharide. K. Kok et al. (Gastroenterology, Vol. 126, no. 4, suppl. 2, pp. A414-A415) describes a study where peptide arrays were used to identify the target of the kinase inhibitor CNI-1493. Therefore kinase activity profiles were made from control cells and cells treated with CNI-1493. Mori et al. (Analytical Biochemistry, Vol. 375, pp. 223-231), provides a study where a peptide microarray comprising substrate peptides for multiple kinases is used to monitor the kinase activity in cell lysates. The study shows how a kinase profile of a A431 human epithelial carcinoma cell lysates can be obtained. Finally, WO 2008/049930 concerns a method for pharmacologically profiling compounds using an array of kinase substrates, immobilized on a porous matrix. The method is found particularly useful in the prediction of drug response, i.e. to enable the distinction between responders and non-responders in the treatment of cells, tissues, organs or warm-blooded animals with the compound to be tested.

The present invention therefore provides a high-throughput and highly sensitive method for monitoring protein phosphorylation and assessing the effect of a specific drug. By acquiring phosphorylation profiles of tissue or cell samples in the absence and in the presence of a specific drug, the method of the present invention can be used to assess the effect of that drug. In particular the present invention is directed to the use of a kinase inhibitor in the method of the present invention to enhance the differentiating capacity of the method.

### Summary of the invention

The present invention is directed to systems and methods for profiling drugs, preferably kinase inhibitors, and particularly to those systems and methods allowing the characterization of the drugs and the prediction of the response of cells, tissues, organs, and/or warm blooded animals to said drugs. The method of the present invention may be employed for diagnostical, prognostical and/or treatment predictive purposes.

The present invention therefore relates to a method for diagnosing, determining the prognosis and/or predicting the treatment outcome of a cancer pathology, said method comprising the steps of:
a) determining kinase activity of one or more clinical tissue lysate or cell lysate samples from a patient by incubating said samples with ATP on an array of two or more protein kinase substrates, thereby generating a kinase activity profile;
b) determining kinase activity of one or more clinical tissue lysate or cell lysate samples from a patient by incubating said samples with a kinase inhibitor and ATP on an array of two or more protein kinase substrates, thereby generating a kinase activity profile;
c) inferring the influence of said kinase inhibitor on the kinase activity profile, whereby an inhibition profile is generated by comparing the kinase activity profiles obtained in step a) and b); and,
d) comparing inhibition profiles obtained from clinical reference tissue lysate or cell lysate samples with the inhibition profile obtained in step c), thereby diagnosing, determining the prognosis and/or predicting the treatment outcome of a cancer pathology.

The present invention further relates to the use of the method of the present invention or a kinase inhibition profile according to the invention, for diagnostical, prognostical, and/or treatment predictive purposes

The present invention also relates in another embodiment to a method wherein said protein kinase substrates are at least two protein kinase substrates selected from the group consisting of the protein kinase substrates with sequence numbers 1 to 157, more particularly using at least two peptides selected from the group consisting of the peptides with sequence numbers 23, 71 and 152.

### Description of the figures

**FIG. 1** shows kinase activity profiles of two tumor cell lines obtained in the absence (A) and in the presence (B) of Gefitinib.
**FIG. 2** shows a virtual experiment explaining the findings of increased discriminatory power of the inhibition profiles in the presence of a protein kinase inhibitor.
**FIG. 3a** shows phosphorylation profiles poorly discriminating treatment responsive, intermediate responsive and non-responsive tumor cells.
**FIG. 3b** shows inhibition profiles discriminating treatment responsive, intermediate responsive and non-responsive tumor cells.
**FIG. 3c** shows inhibition profiles of three selected peptides.
**FIG 4** shows an example of the reduced variance in inhibition profiles versus basal phosphorylation profiles.

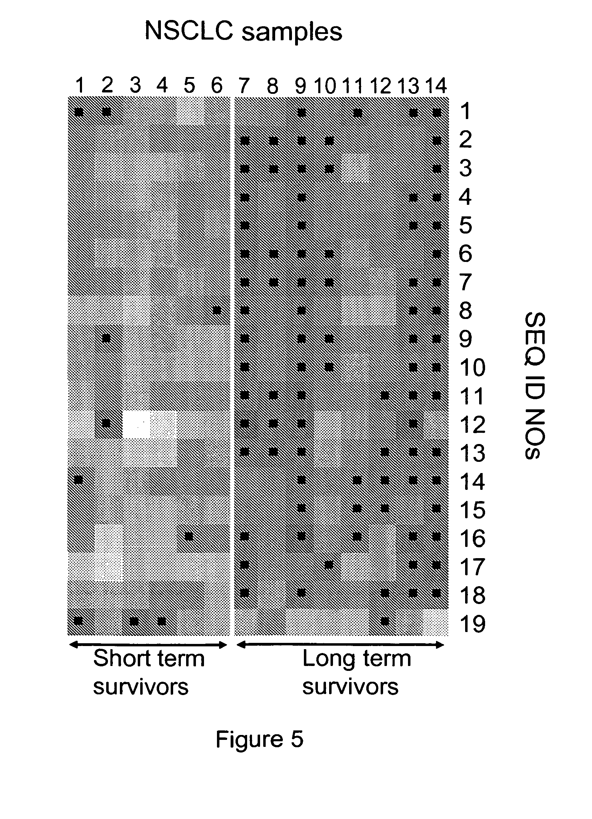

**FIG 5** provides a heatmap as depicted in the examples representing the ratio of inhibited versus not inhibited phosphorylation signals for each of the peptide markers listed in Table 3.

### Detailed description

Before the present method and devices used in the invention are described, it is to be understood that this invention is not limited to particular methods, components, or devices described, as such methods, components, and devices may, of course, vary. It is also to be understood that the terminology used herein is not intended to be limiting.

Unless defined otherwise, all technical and scientific terms used herein have the same meaning as commonly understood by one of ordinary skill in the art to which this invention belongs. Although any methods and materials similar or equivalent to those described herein may be used in the practice or testing of the present invention, the preferred methods and materials are now described.

In this specification and the appended claims, the singular forms "a", "an", and "the" include plural references unless the context clearly dictates otherwise.

The present invention provides a highly sensitive and high-throughput method where protein phosphorylation is monitored under specific circumstances. By comparing the protein phosphorylation in the absence and in the presence of a certain kinase inhibitor, the mechanism of action of that kinase inhibitor can be measured directly thereby assessing the effect of that specific kinase inhibitor.

By 'kinase inhibitor' we refer to a type of enzyme inhibitor which specifically blocks the action of a kinase. A kinase inhibitor is a molecule that binds a kinase and decreases its activity. The binding of the kinase inhibitor can stop a substrate from entering the active site of the kinase and/or hinder the kinase from catalyzing its reaction. Kinase inhibitors are used in the treatment of cancer. The present invention especially refers to protein kinase inhibitors, enzyme inhibitors that specifically block the action of protein kinases.

Protein kinase activity is referred to as the activity of protein kinases. A protein kinase is a kinase enzyme that modifies other proteins by chemically adding phosphate groups to them. This process or activity is also referred to as phosphorylation. Phosphorylation usually results in a functional change of the substrate by changing enzyme activity, cellular location, or association with other proteins. Up to 30% of all proteins may be modified by kinase activity, and kinases are known to regulate the majority of cellular pathways, especially those involved in signal transduction, the transmission of signals within the cell. The chemical activity of a kinase involves removing a phosphate group from ATP, or any other phosphate source, and covalently attaching it to amino acids such as serine, threonine, tyrosine, histidine, aspartatic acid and/or glutamic acid that have a free hydroxyl group. Most known kinases act on both serine and threonine, others act on tyrosine, and a number act on all serine, threonine and tyrosine. The protein kinase activity monitored with the method of the present invention is preferably directed to protein kinases acting towards serine, threonine and/or tyrosine, preferably acting on both serine and threonine, on tyrosine or on serine, threonine and tyrosine.

Because protein kinases have profound effects on a cell, their activity is highly regulated. Kinases are turned on or off by for instance phosphorylation, by binding of activator proteins or inhibitor proteins, or small molecules, or by controlling their location in the cell relative to their substrates. Deregulated kinase activity is a frequent cause of disease, particularly cancer, where kinases regulate many aspects that control cell growth, movement and death. Therefore monitoring the protein kinases are often the target of for therapeutic intervention.

Accordingly, within one embodiment of the present invention, a method is provided for diagnosing, determining the prognosis and/or predicting the treatment outcome of a cancer pathology, said method comprising the steps of:
a) determining kinase activity of one or more clinical tissue lysate or cell lysate samples from a patient by incubating said samples with ATP on an array of two or more protein kinase substrates, thereby generating a kinase activity profile;
b) determining kinase activity of one or more clinical tissue lysate or cell lysate samples from a patient by incubating said samples with a kinase inhibitor and ATP on an array of two or more protein kinase substrates, thereby generating a kinase activity profile;
c) inferring the influence of said kinase inhibitor on the kinase activity profile, whereby an inhibition profile is generated by comparing the kinase activity profiles obtained in step a) and b); and,
d) comparing inhibition profiles obtained from clinical reference tissue lysate or cell lysate samples with the inhibition profile obtained in step c), thereby diagnosing, determining the prognosis and/or predicting the treatment outcome of a cancer pathology.

In a preferred embodiment of the present invention the inhibition profile can be determined by comparing the kinase activity profiles obtained in steps a) and b) of the method of the present invention with other kinase activity profiles obtained from other samples. The kinase activity profiles from other samples can for instance be kinase activity profiles obtained from earlier conducted tests.

In a preferred embodiment, three or more different samples are compared in steps a) and b) in the method of the present invention. A comparison of three or more different samples renders the method of the present invention more robust and more precise. When for instance the activity profile of a sample is compared to a large set of activity profiles from a database, the method of the present invention will be more specific and precise.

The substrates as used herein, are meant to include hormone receptors, peptides, proteins and/or enzymes. In particular the substrates used are kinase substrates, more in particular peptide kinase substrates, even more particular the peptide kinase substrates in Table 1 and/or Table 3, most particularly using at least 2, 3, 4, 5, 9, 10, 12, 16, 19, 20, 30, 40, 50, 60, 70, 80, 90, 100, 110, 120, 130, 140, 144, 150 or 157 peptides of the peptide kinase substrates in Table 1 and/or Table 3. In a preferred embodiment the array of substrates comprises at least two peptides selected from the group consisting of the peptides with any of Seq.Id.No. 23, 71 and 152. In a more preferred embodiment the array of substrates comprises or consists of the peptides with any of Seq.Id.No. 23, 71 and 152.

It should be noted that a person skilled in the art will appreciate that the kinase substrates used in the methods of the present invention and immobilized on the arrays of the invention may be the peptides as listed in Table 1 and/or Table 3. These peptides can be used according to the methods or arrays of the present invention to measure the phosphorylation levels of phosphorylation sites of said peptides in the presence of protein kinase present in the samples. The phosphorylation levels of the individual phosphorylation sites present in said peptides may be measured and compared in different ways. Therefore the present invention is not limited to the use of peptides identical to any of the peptides as listed in Table 1 and/or Table 3 as such. The skilled person may easily on the basis of the sequence of the peptides listed in Table 1 and/or Table 3 design variants compared to the specific peptides in said tables and use such variants in a method for measuring phosphorylation levels of phosphorylation sites present in said peptides as listed in Table 1 and/or Table 3. These variants may be peptides which have a one or more (2, 3, 4, 5, 6, 7, etc.) amino acids more or less than the given peptides and may also have amino acid substitutions (preferably conservative amino acid substitutions) as long as these variant peptides retain at least one or more of the phosphorylation sites of said original peptides as listed in said tables. Further the skilled person may also easily carry out the methods or construct arrays according to the present invention by using proteins (full length or N- or C-terminally truncated) comprising the amino acid regions of the peptides listed in Table 1 and/or Table 3 as sources for studying the phosphorylation of sites present in the amino acid regions of the peptides listed in Table 1 and/or Table 3. Also the skilled person may use peptide mimetics which mimick the peptides listed in Table 1 and/or Table 3. The present invention also includes the use of analogs and combinations of these peptides for use in the method or arrays according to the present invention. The peptide analogs include peptides which show a sequence identity of more than 70%, preferably more than 80% and more preferably more than 90%.

As used herein "peptide" refers to a short truncated protein generally consisting of 2 to 100, preferably 2 to 30, more preferably 5 to 30 and even more preferably 13 to 18 naturally occurring or synthetic amino acids which can also be further modified including covalently linking the peptide bonds of the alpha carboxyl group of a first amino acid and the alpha amino group of a second amino acid by eliminating a molecule of water. The amino acids can be either those naturally occurring amino acids or chemically synthesized variants of such amino acids or modified forms of these amino acids which can be altered from their basic chemical structure by addition of other chemical groups which can be found to be covalently attached to them in naturally occurring compounds.

As used herein "protein" refers to a polypeptide made of amino acids arranged in a linear chain and joined together by peptide bonds between the carboxyl and amino groups of adjacent amino acid residues.

As used herein "peptide mimetics" refers to organic compounds which are structurally similar to peptides and similar to the peptide sequences list in Table 1 and/or Table 3. The peptide mimetics are typically designed from existing peptides to alter the molecules characteristics. Improved characteristics can involve, for example improved stability such as resistance to enzymatic degradation, or enhanced biological activity, improved affinity by restricted preferred conformations and ease of synthesis. Structural modifications in the peptidomimetic in comparison to a peptide, can involve backbone modifications as well as side chain modification.

**Table 1: list of 157 peptides used for determining the kinase activity, their sequence and Seq.Id.No.**

| Seq.Id.No. | Peptide name | Peptide amino acid sequence |
|---|---|---|
| 1 | 41_348_660_Y354 | LDGENIYIRHSNL |
| 2 | 41_653_665_Y627 | RLDGENIYIRHSN |
| 3 | ACHB_383_395_Y390 | WGRGTDEYFIRKP |
| 4 | ACHD_383_395_Y390 | YISKAEEYFLLKS |
| 5 | AMPE_5_17_Y12 | EREGSKRYCIQTK |
| 6 | ANXA1_13_25_Y20/T23 | IENEEQEYVQTVK |
| 7 | ANXA2_16_28_T18/S17/S21/S25/Y23 | HSTPPSAYGSVKA |
| 8 | ART_004_EAIYAAPFAKKKXC | EAIYAAPFAKKK |
| 9 | B3AT_39_51_Y46/S50 | TEATATDYHTTSH |
| 10 | C1R_199_211_S206 | TEASGYISSLEYP |
| 11 | CALM_93_105_Y99/S101 | FDKDGNGYISAAE |
| 12 | CALM_95_107_Y99/S101 | KDG NGYISAAELR |
| 13 | CBL_693_705_Y700 | EGEEDTEYMTPSS |
| 14 | CD3Z_116_128_Y123 | KDKMAEAYSEIGM |
| 15 | CD3Z_146_158_Y153 | STATKDTYDALHM |
| 16 | CD79A_181_193_Y182/Y188 | EYEDENLYEGLNL |
| 17 | CDK2_8_20_T14/Y15 | EKIGEGTYGVVYK |
| 18 | CDK7_157_169_S164 | GLAKSFGSPNRAY |
| 19 | CREB1_122_134_Y134/S133 | QKRREILSRRPSY |
| 20 | CRK_214_226_Y221 | GPPEPGPYAQPSV |
| 21 | CTNB1_79_91_Y86 | VADIDGQYAMTRA |
| 22 | DCX_109_121_Y112/S116 | GIVYAVSSDRFRS |
| 23 | DDR1_506_518_Y513 | LLLSNPAYRLLLA |
| 24 | DDR1_785_797_Y792/Y796/Y797 | FGMSRNLYAGDYY |
| 25 | DDR2_733_745_Y740 | RNLYSGDYYRIQG |
| 26 | DYR1A_212_224_Y219 | KHDTEMKYYIVHL |
| 27 | DYR1A_312_324_Y319/Y321 | CQLGQRIYQYIQS |
| 28 | EFS_246_258_Y253 | GGTDEGIYDVPLL |
| 29 | EFS_246_258_Y253F | GGTDEGIFDVPLL |
| 30 | EGFR_1062_1074_Y1069 | EDSFLQRYSSDPT |
| 31 | EGFR_1103_1115_Y1110 | GSVQNPVYHNQPL |
| 32 | EGFR_1118_1130_Y1125 | APSRDPHYQDPHS |
| 33 | EGFR_1165_1177_Y1172 | ISLDNPDYQQDFF |
| 34 | EGFR_1190_1202_Y1197 | STAENAEYLRVAP |
| 35 | EG_FR_862_874_Y869 | LGAEEKEYHAEGG |
| 36 | EGFR_908_920_Y915 | MTFGSKPYDGIPA |
| 37 | ENOG_37_49_Y43 | SGASTGIYEALEL |
| 38 | EPHA1_774_786_Y781 | LDDFDGTYETQGG |
| 39 | EPHA2_581_593_Y588 | QLKPLKTYVDPHT |
| 40 | EPHA2_765_777_Y772 | EDDPEATYTTSGG |
| 41 | EPHA4_589_601_Y596 | LNQGVRTYVDPFT |
| 42 | EPHA4_921_933_Y928 | QAIKMDRYKDNFT |
| 43 | EPHA7_607_619_Y608/Y614 | TYIDPETYEDPNR |
| 44 | EPH_B1_771_783_Y778 | DDTSDPTYTSSLG |
| 45 | EPHB1_921_933_Y928 | SAIKMVQYRDSFL |
| 46 | EPHB4_583_595_Y590 | IGHGTKVYIDPFT |
| 47 | EPOR_361_373_Y368 | SEHAQDTYLVLDK |
| 48 | EPOR_419_431_Y426 | ASAASFEYTILDP |
| 49 | ERBB2_1241_1253_Y1248 | PTAENPEYLGLDV |
| 50 | ERBB2_870_882_Y877 | LDIDETEYHADGG |
| 51 | ERBB2_945_957_Y952 | PISTIDVYMIMVK |
| 52 | ERBB4_1181_1193_Y1188 | QALDNPEYHNASN |
| 53 | ERBB4_1277_1289_Y1284 | IVAENPEYLSEFS |
| 54 | F261_26_38_S33 | RLQRRRGSSIPQF |
| 55 | FABH_13_25_Y19 | DSKNFDDYMKSLG |
| 56 | FAK1_569_581_Y576/Y577 | RYMEDSTYYKASK |
| 57 | FAK2_572_584_Y579/Y580 | RYIEDEDYYKASV |
| 58 | FER_707_719_Y714 | RQEDGGVYSSSGL |
| 59 | FES_706_718_Y713 | REEADGVYAASGG |
| 60 | FGFR1_759_771_Y766 | ALTSNQEYLDLSM |
| 61 | FGFR2_762_774_Y769 | TLTTNEEYLDLSQ |
| 62 | FGFR3_641_653_Y648 | DVHNLDYYKKTTN |
| 63 | FGFR3_753_765_Y760 | TVTSTDEYLDLSA |
| 64 | FRK_380_392_Y387 | KVDNEDIYESRHE |
| 65 | GSK3B_209_221_Y216 | RGEPNVSYICSRY |
| 66 | H2BR_26_38_S32/S36 | DGKKRKRSRKESY |
| 67 | INSR_1348_1360_S1354/Y1355 | SLGFKRSYEEHIP |
| 68 | INSR_993_1005_Y993/Y999 | YASSNPEYLSASD |
| 69 | IRS1_1222_1234_Y1230 | SSEDLSAYASISF |
| 70 | IRS2_535_545_Y540 | GGGGGEFYGYMTM |
| 71 | JAK1_1015_1027_Y1022/Y1023 | AIETDKEYYTVKD |
| 72 | K2C6E_53_65_S59 | GAGFGSRSLYGLG |
| 73 | K2C8_425_437_S431 | SAYGGLTSPGLSY |
| 74 | KSYK_518_530_Y525/Y526 | ALRADENYYKAQT |
| 75 | LAT_194_206_Y200 | MESIDDYVNVPES |
| 76 | LAT_249_261_Y255 | EEGAPDYENLQEL |
| 77 | LCK_387_399_Y394 | RLIEDNEYTAREG |
| 78 | LTK_669_681_Y772/Y776/Y777 | RDIYRASYYRRGD |
| 79 | MBP_198_210_Y203 | ARTAHYGSLPQKS |
| 80 | MBP_259_271_Y261/Y268/S266 | FGYGGRASDYKSA |
| 81 | MBP_263_275_Y268/S266/S270 | GRASDYKSAHKGF |
| 82 | MET_1227_1239_Y1230/Y1234/Y1235 | RDMYDKEYYSVHN |
| 83 | MK01_180_192_Y187 | HTGFLTEYVATRW |
| 84 | MK01_198_210_Y205 | IMLNSKGYTKSID |
| 85 | MK07_211_223_T218/Y220 | AEHQYFMTEYVAT |
| 86 | MK10_216_228_T221/Y223 | SFMMTPYVVTRY |
| 87 | MK12_178_190_T183/Y185 | ADSEMTGYVVTRW |
| 88 | MK14_173_185_T180/Y182 | RHTDDEMTGYVAT |
| 89 | NCF1_313_325_S315/S320 | QRSRKRLSQDAYR |
| 90 | NPT2_501_513_T508 | AKALGKRTAKYRW |
| 91 | NTRK1_489_501_Y496 | HIIENPQYFSDAC |
| 92 | NTRK2_509_521_Y516 | PVIENPQYFGITN |
| 93 | NTRK2_695_707_Y702/Y706/Y707 | FGMSRDVYSTDYY |
| 94 | NTRK2_699_711_Y702/Y706/Y707 | RDVYSTDYYRVGG |
| 95 | ODBA_340_352_S345 | DDSSAYRSVDEVN |
| 96 | ODPAT_291_303_S291/S293 | SMSDPGVSYRTRE |
| 97 | P2AB_297_309_T304/Y307 | EPHVTRRTPDYFL |
| 98 | P85A_600_612_Y607/S608 | NENTEDQYSLVED |
| 99 | PAXI_111_123_Y118 | VGEEEHVYSFPNK |
| 100 | PAXI_24_36_Y31 | FLSEETPYSYPTG |
| 101 | PDPK1_2_14_Y9 | ARTTSQLYDAVPI |
| 102 | PDPK1_369_381_Y373/Y376 | DEDCYGNYDNLLS |
| 103 | PECA1_706_718_Y713 | KKDTETVYSEVRK |
| 104 | PERI_459_471_Y471 | QRSELDKSSAHSY |
| 105 | PGFRB_1002_1014_Y1009 | LDTSSVLYTAVQP |
| 106 | PGFRB_572_584_Y579/Y581 | VSSDGHEYIYVDP |
| 107 | PGFRB_709_721_Y716 | RPPSAELYSNALP |
| 108 | PGFRB_768_780_Y771/Y775/Y778 | SSNYMAPYDNYVP |
| 109 | PGFRB_771_783_Y771/Y775/Y778 | YMAPYDNYVPSAP |
| 110 | PLCG1_1246_1258_S1248/Y1253 | EGSFESRYQQPFE |
| 111 | PLCG1_764_776_Y771 | IGTAEPDYGALYE |
| 112 | PLCG1_776_788_Y783 | EGRNPGFYVEANP |
| 113 | PRGR_545_557_S552 | LRPDSEASQSPQY |
| 114 | PRGR_786_798_S793 | EQRMKESSFYSLC |
| 115 | PRRX2_202_214_Y214 | WTASSPYSTVPPY |
| 116 | PTN11_535_547_Y542 | SKRKGHEYTNIKY |
| 117 | RAF1_331_343_S337/S338/Y339/Y340 | RPRGQRDSSYYWE |
| 118 | RASA1_453_465_Y460 | TVDGKEIYNTIRR |
| 119 | RB_804_816_S807/S811 | IYISPLKSPYKIS |
| 120 | RBL2_99_111_Y111/S103 | VPTVSKGTVEGNY |
| 121 | RET_1022_1034_Y1029 | TPSDSLIYDDGLS |
| 122 | RET_680_692_Y687 | AQAFPVSYSSSGA |
| 123 | RON_1346_1358_Y1353 | SALLGDHYVQLPA |
| 124 | RON_1353_1365_Y1356/Y1360 | YVQLPATYMNLGP |
| 125 | SRC8_CHICK_470_482_Y477 | VSQREAEYEPETV |
| 126 | SRC8_CHICK_476_488_Y477/Y483 | EYEPETVYEVAGA |
| 127 | SRC8_CHICK_492_504_Y499 | YQAEENTYDEYEN |
| 128 | STA5A_687_699_Y694 | LAKAVDGYVKPQI |
| 129 | STAT1_694_706_Y701 | DGPKGTGYIKTEL |
| 130 | STAT2_683_695_Y690 | NLQERRKYLKHRL |
| 131 | STAT3_698_710_Y705 | DPGSAAPYLKTKF |
| 132 | STAT4_686_698_Y693 | TERGDKGYVPSVF |
| 133 | STAT4_714_726_Y725 | PSDLLPMSPSVYA |
| 134 | STAT6_634_646_Y641 | MGKDGRGYVPATI |
| 135 | SYN1_2_14_S9 | NYLRRRLSDSNFM |
| 136 | TAU_512_524_Y514/T522 | SGYSSPGSPGTPG |
| 137 | TEC_512_524_Y519 | RYFLDDQYTSSSG |
| 138 | TNNT1_2_14_Y9 | SDTEEQEYEEEQP |
| 139 | TYRO3_679_691_Y686 | KIYSGDYYRQGCA |
| 140 | VEGFR1_1040_1052_Y1048 | DFGLARDIYKNPD |
| 141 | VEGFR1_1046_1058_Y1053/Y148F | DIFKNPDYVRKGD |
| 142 | VEGFR1_1049_1061_Y1053 | KNPDYVRKGDTRL |
| 143 | VEGFR1_1162_1174_Y1169 | VQQDGKDYIPINA |
| 144 | VEGFR1_1206_1218_Y1213 | GSSDDVRYVNAFK |
| 145 | VEG_FR1_1235_1247_Y1242 | ATSMFDDYQGDSS |
| 146 | VEGFR1_1320_1332 Y1327/C1320S/C1321S | SSSPPPDYNSVVL |
| 147 | VEGFR1_1326_1338_Y1333 | DYNSVVLYSTPPI |
| 148 | VEGFR2_1046_1058_Y1054 | DFGLARDIYKDPD |
| 149 | VEGFR2_1052_1064_Y1059 | DIYKDPDYVRKGD |
| 150 | VEGFR2_1068_1080_Y1175 | AQQDGKDYIVLPI |
| 151 | VEGFR2_1207_1219_Y1214/C1208S | VSDPKFHYDNTAG |
| 152 | VEGFR2_944_956_Y951 | RFRQGKDYVGAIP |
| 153 | VEGFR2_989_1001_Y996 | EEAPEDLYKDFLT |
| 154 | VGFR3_1061_1073_Y1063/Y1068/S1073 | DIYKDPDYVRKGS |
| 155 | VINC_815_827_Y821 | KSFLDSGYRILGA |
| 156 | ZAP70_485_497_Y492/Y493 | ALGADDSYYTARS |
| 157 | ZBT16_621_633_S628 | LRTHNGASPYQCT |

The term 'tissue or cell samples' as used herein, refers to samples obtained from an organism such as human, plant, bacteria, fungi, etc., or from components (e.g., cells) of such an organism. The sample could in principle be any biological sample, such as for example blood, urine, saliva, tissue biopsy or autopsy material and then in particular cell lysates thereof, but would typically consist of cell lysates prepared from cell lines, including cancer cell lines; primary and immortalized tissue cell lines; non-human animal model biopsies and patient biopsies. In one embodiment of the invention, the cell lysates are prepared from cancer cell lines; xenograft tumors or cancer patient biopsies, including tumor and normal tissue. Frequently a sample will be a 'clinical sample' which is a sample derived from a patient. Such samples include, but are not limited to, sputum, blood, blood fractions such as serum including fetal serum (e.g., SFC) and plasma, blood cells (e.g., white cells), tissue or fine needle biopsy samples, urine, peritoneal fluid, and pleural fluid, or cells there from.

The tissue samples may also refer to surrogate tissues. The ideal tissue to perform pharmacodynamic studies is the own tumor. However, taking in consideration the difficulties to perform sequential tumor biopsies, surrogate tissues can be used instead. Therefore, a distant tissue, such as skin tissue, can be used as a surrogate tissue for a cancerous tissue. The surrogate tissue can be used to monitor, or predict the effects of a drug. For example skin and hair tissue are known for their use as a prediction for the response of tumors to treatment with signalling inhibitors.

Since kinase inhibitors are often used in the treatment of cancer the present invention relates to a method wherein a tissue or fluid sample is provided from a patient, the sample containing tumor cells.

The samples can either be obtained from healthy as well as diseased sources.

In a preferred embodiment of the present invention a diseased tissue or cell sample is a tumor tissue or cell sample. The tumor tissue or cell sample can be obtained from any cancer known in the art and for instance chosen from the group comprising brain cancer, breast cancer, prostate cancer, ovarian cancer, colon cancer, endometrium cancer, lung cancer, bladder cancer, stomach cancer, osteophagus cancer, oral tongue cancer, oral cavity cancer, skin cancer, mesotheliomas, retinoblastomas, and/or nephroblastomas and more preferably brain cancer, breast cancer, ovarian cancer and/or colon cancer.

In a preferred embodiment of the present invention the tissue or cell sample is a healthy tissue or cell sample. The method of the present invention using a healthy tissue or cell sample instead of a diseased tissue or cell sample can be used as a reference to assess the safety of a specific drug. The inhibition profile obtained by the method of the present invention using healthy tissue or cell samples can provide significant information concerning the safety of the drug since information is obtained as to which signalling pathways are targeted by the drug. Healthy tissue or cell samples can be taken from any known of tissue.

The tissue or cell samples used in the preferred method of the present invention can be pretreated. The pretreatment of the tissue or cell samples depends on the particular compound to be tested, and the type of sample used. The optimum method can be readily determined by those skilled in the art using conventional methods and in view of the information set out herein. Preferably, the tumor tissue or cell samples are lysates. For example, the tissue sample is obtained by lysing the tumor tissue in a particular buffer comprising phosphatases and protease inhibitors.

The tissue or cell samples show a particular enzymatic activity such as for instance a kinase activity due to the protein kinases present in the tissue or cells. Therefore, contacting the samples with an array of two or more substrates and preferably kinase substrates, and more in particular peptide kinase substrates, in the presence of ATP will lead to a phosphorylation of the kinase substrates. This response of the kinase substrates, also referred to as the kinase activity profile of that sample, can be determined using a detectable signal. The signal is the result from the interaction of the sample with the array of substrates. The response of the array of substrates can be monitored using any method known in the art. The response of the array of substrates is determined using a detectable signal, said signal resulting from the interaction of the sample with the array of substrates. As mentioned hereinbefore, in determining the interaction of the sample with the array of substrates the signal is either the result of a change in a physical or chemical property of the detectably labeled substrates, or indirectly the result of the interaction of the substrates with a detectably labeled molecule capable of binding to the substrates. For the latter, the molecule that specifically binds to the substrates of interest (e.g., antibody or polynucleotide probe) can be detectably labeled by virtue of containing an atom (e.g., radionuclide), molecule (e.g., fluorescein), or complex that, due to a physical or chemical property, indicates the presence of the molecule. A molecule may also be detectably labeled when it is covalently bound to or otherwise associated with a "reporter" molecule (e.g., a biomolecule such as an enzyme) that acts on a substrate to produce a detectable atom, molecule or other complex.

Detectable labels suitable for use in the present invention include any composition detectable by spectroscopic, photochemical, biochemical, immunochemical, electrical, optical or chemical means. Labels useful in the present invention include biotin for staining with labeled avidin or streptavidin conjugate, magnetic beads (e.g., Dynabeads'), fluorescent dyes (e.g., fluorescein, fluorescein-isothiocyanate (FITC), Texas red, rhodamine, green fluorescent protein, enhanced green fluorescent protein and related proteins with other fluorescence emission wavelengths, lissamine, phycoerythrin, Cy2, Cy3, Cy3.5, Cy5, Cy5.5, Cy7, FluorX [Amersham], SYBR Green I & II [Molecular Probes], and the like), radiolabels (e.g., 3H,125I, 35S, 4C, or 32P), enzymes (e.g., hydrolases, particularly phosphatases such as alkaline phosphatase, esterases and glycosidases, or oxidoreductases, particularly peroxidases such as horse radish peroxidase, and the like), substrates, cofactors, inhibitors, chemilluminescent groups, chromogenic agents, andcolorimetric labels such as colloidal gold or colored glass or plastic (e. g., polystyrene, polypropylene, latex, etc.) beads.

Means of detecting such labels are well known to those of skill in the art. Thus, for example, chemiluminescent and radioactive labels may be detected using photographic film or scintillation counters, and fluorescent markers may be detected using a photodetector to detect emitted light (e.g., as in fluorescence-activated cell sorting). Enzymatic labels are typically detected by providing the enzyme with a substrate and detecting a colored reaction product produced by the action of the enzyme on the substrate. Colorimetric labels are detected by simply visualizing the colored label. Thus, for example, where the label is a radioactive label, means for detection include a scintillation counter, photographic film as in autoradiography, or storage phosphor imaging. Where the label is a fluorescent label, it may be detected by exciting the fluorochrome with the appropriate wavelength of light and detecting the resulting fluorescence. The fluorescence may be detected visually, by means of photographic film, by the use of electronic detectors such as charge coupled devices (CCDs) or photomultipliers and the like. Similarly, enzymatic labels may be detected by providing the appropriate substrates for the enzyme and detecting the resulting reaction product. Also, simple colorimetric labels may be detected by observing the color associated with the label. Fluorescence resonance energy transfer has been adapted to detect binding of unlabeled ligands, which may be useful on arrays.

In a particular embodiment of the present invention the response of the array of substrates to the sample is determined using detectably labeled antibodies; more in particular fluorescently labeled antibodies. In those embodiments of the invention where the substrates consist of kinase substrates, the response of the array of substrates is determined using fluorescently labeled anti-phosphotyrosine antibodies, fluorescently labeled anti-phosphoserine or fluorescently labeled anti-phosphoserine antibodies. As outlined in more detail in the examples hereinafter, the use of fluorescently labeled anti-phosphotyrosine antibodies or fluorescently labeled anti-phosphoserine or fluorescently labeled anti-phosphoserine antibodies allows real-time or semi real-time determination of the substrate activity and accordingly provides the possibility to express the array activity as the initial kinase velocity.

In a preferred embodiment of the present invention, the response of the array of kinase substrates is determined using fluorescently labeled anti-phosphotyrosine or fluorescently labeled anti-phosphoserine or fluorescently labeled anti-phosphoserine antibodies antibodies. Furthermore, the use of fluorescently labeled anti-phosphotyrosine antibodies or fluorescently labeled anti-phosphoserine or fluorescently labeled anti-phosphoserine antibodies do not prevent real-time or semi real-time determination of the substrate activity and accordingly provides the possibility to express the array activity as the initial kinase velocity

The presence of a drug, and more preferably a kinase inhibitor, during the measurement of the kinase activity will result in an inactivation of certain protein kinases active in the tissue or cell sample. Therefore a comparison of the kinase activity profiles in the presence and in the absence of a drug will provide a new profile or inhibition profile which provides important information on the activity of the drug.

In a preferred embodiment of the present invention said drug is a kinase inhibitor. In another embodiment of the present invention said drug is a protein phosphatase inhibitor.

In a preferred embodiment of the present invention the method further comprises the presence of one or more protein kinase inhibitor in steps a) and b).

In another embodiment the method further comprises the presence one or more protein phosphatases in steps a) and b). By providing one or more protein kinase inhibitors or one or more protein phosphatases in the steps where the kinase activity of the tissue or cell samples is determined, it was surprisingly shown that the presence of the protein kinase inhibitor or protein phosphatase resulted in a better differentiation between the kinase activity of the tissue or cell samples in the presence and in the absence of said drug. This results in a more efficient differentiation between the kinase activity profiles and a clearer inhibition profile. The clearer differentiation obtained when the experiments are conducted in the presence of a kinase inhibitor are due to the fact that protein kinases have a promiscuous activity and the addition of a kinase inhibitor during the measurement can alter the kinase activity profile of a sample, thereby rendering the difference between the kinase activity profiles in the absence and in the presence of a drug more significant.

The array of substrates is preferably a microarray of substrates wherein the substrates are immobilized onto a solid support or another carrier. The immobilization can be either the attachment or adherence of two or more substrate molecules to the surface of the carrier including attachment or adherence to the inner surface of said carrier in the case of e.g. a porous or flow-through solid support.

In a preferred embodiment of the present invention, the array of substrates is a flow-through array. The flow-through array as used herein could be made of any carrier material having oriented through-going channels as are generally known in the art, such as for example described in PCT patent publication WO 01/19517. Typically the carrier is made from a metal oxide, glass, silicon oxide or cellulose. In a particular embodiment the carrier material is made of a metal oxide selected from the group consisting of zinc oxide, zirconium oxide, tin oxide, aluminium oxide, titanium oxide and thallium; in a more particular embodiment the metal oxide consists of aluminium oxide.

In a preferred embodiment of the present invention, the substrates are at least 2, 3, 4, 5, 9, 10, 12, 16, 19, 20, 30, 40, 50, 60, 70, 80, 90, 100, 110, 120, 130, 140, 144, 150 or 157 protein kinase substrates used in the methods and arrays of the present invention are selected from the group consisting of the protein kinase substrates with any of Seq.Id.No.1 to 157, most particularly using at least two peptides selected from the group consisting of the peptides with any of Seq.Id.No. 23, 71 and/or 152. In a more preferred embodiment the substrates are the peptides with any of Seq.Id.No. 23, 71 and/or 152.

The present invention also discloses a kinase inhibition profile obtained by the method of the present invention. The kinase inhibition profile thereby being specific for a certain pathology. Potential pathologies include, but are not limited to, oncological diseases, metabolic diseases, immunological and auto-immunological diseases, diseases of the nervous system and/or infectious diseases.

The present invention also relates to a computer platform comprising reference kinase activity profiles and/or reference inhibition profiles from tissue and cell samples and comparing the kinase activity profiles and/or inhibition profiles of the tissue and cell samples analysed using the method of the present invention with said reference profiles. The computer program can be provided on a data carrier comprising reference kinase activity profiles and/or reference inhibition profiles. Said computer program would enable the prediction of the response of cells, tissues, organs and/or warm-blooded animals to said drug. Furthermore, said computer program can be used for diagnostical purposes, prognostical purposes, for the prediction of the clinical outcome of a therapy, for treatment predictive purposes, for predicting and determining side effects and/or toxic effects and/or adverse effects of a therapy

Furthermore, the present application discloses a kinase inhibition profile obtained by the method of the present invention, wherein said kinase inhibition profile can be used for diagnostical and/or prognostical purposes and/or for the prediction of the clinical outcome of a therapy, and/or of side effects and/or toxic effects and/or adverse effects of a therapy. For example the method of the present invention can be used to diagnose a cancer and preferably brain cancer, thereby differentiating between benign and malignant tumors.

In another embodiment, the present invention relates to a method of the invention or array as disclosed, the use of a method according to the invention or a kinase inhibition profile according to the invention, for the prediction of the response of cells, tissues, organs and/or warm-blooded animals to said drug.

In another embodiment, the present invention relates to a method of the invention or array as disclosed, the use of a method according to the invention or a kinase inhibition profile according to the invention, for diagnostical, prognostical, and/or treatment predictive purposes. The kinase inhibition profiles can for instance be used to assess the likelihood of a particular favourable or unfavourable outcome, susceptibility (or lack thereof) to a particular therapeutic regimen, etc.

In yet another embodiment, the present invention relates to a method according to the present invention, the use of a method according to the invention or a kinase inhibition profile as disclosed, for determining the clinical outcome of a drug therapy in cancer.

As disclosed, the inhibition profile is generated using an array of substrates for carrying out the method of the present invention comprising at least two peptides selected from the peptide kinase substrates with any of Seq.Id.No. 1 to 157, most particularly using at least two peptides selected from the group consisting of the peptides with any of Seq.Id.No. 23, 71 and 152. In a more preferred embodiment the substrates are the peptides with any of Seq.Id.No. 23, 71 and 152.

The present application further discloses an array of substrates for carrying out the method of the present invention comprising at least two protein kinase substrates selected from the group consisting of the protein kinase substrates with any of Seq.Id.No. 1 to 157, most particularly using at least two peptides selected from the group consisting of the peptides with any of Seq.Id.No. 23, 71 and 152. In a more preferred embodiment the substrates are the peptides with any of Seq.Id.No. 23, 71 and 152.

Furthermore, the method of the present invention can be used for assessing the pharmaceutical value of a drug and/or the clinical value of a drug.

In a further embodiment, the present application discloses a kit offering the necessary components for performing the method of the present invention.

### Examples

### Example 1:

In order to assess the effect of the presence of a kinase inhibitor during the determination of the kinase activity, the kinase activity profile of samples of the tumor cell line HCC827 and the Gefitinib resistant HCC827 R cell line are monitored in the presence and in the absence of a kinase inhibitor Gefitinib. Gefitinib is a selective inhibitor of epidermal growth factor receptor's (EGFR) tyrosine kinase domain.

Cells were harvested and were lysed in Mammalian Extraction Buffer (M-PER, Pierce) containing phosphatase and protease inhibitors (HALT, Pierce). Five microliter of the lysis solution was pipetted into a reaction mixture composed of 1x ABL buffer (New England Biolabs, cat.nr B6050S), 0,1% Bovine Serum Albumin, 100 µM ATP, 20 µg/ml phosphotyrosine antibody to an end volume of 40 microliter. Before incubation a blocking step was carried out on the substrate arrays with 0.2% bovine serum albumin. After loading of the reaction mixtures into the Pamchip arrays comprising 144 peptides (kinase substrates) each, incubation was commenced thereby measuring the kinase activity of each sample. Real time data were obtained by measuring fluorescence of the bound anti-phosphotyrosine antibody after each 5 cycles. Image quantification and data processing was conducted with dedicated Pamgene software (Evolve and Bionavigator). Subsequent data analysis was performed using Genespring (Agilent) and Excel (Microsoft).

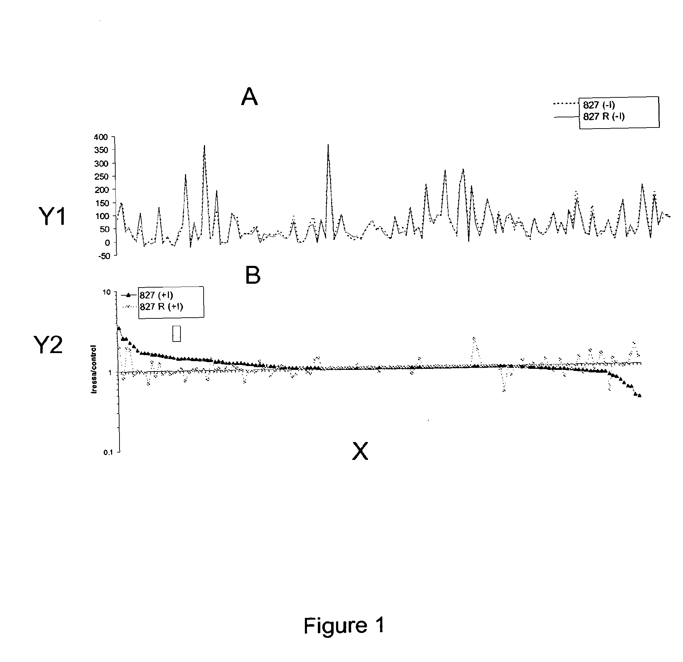

Figure 1 shows the kinase activity profiles obtained in the absence (A) and in the presence (B) of Gefitinib. The annotation of figure 1 are: X = Different peptides on the pamchip microarray; Y1 = fluorescence intensity representing peptide phosphorylation (AU); Y2 = ratio of phosphorylation signal (inhibition) in presence and absence of kinase inhibitor (I) Gefitinib (also referred to as Iressa), thus signals are normalized to median of signal in absence of inhibitor, only DMSO solvent).

In the absence (A) of Gefitinib the kinase profiles of the HCC827 (dotted line) and HCC827 R (full line) tumor cell lines are almost identical while the presence of Gefitinib (B) during the measurement of the kinase activity profile the ratio of the kinase activity over the control value shows a clear difference between the HCC827 (black triangles) and HCC827 R (grey circles) tumor cell lines.

This clearly shows that the presence of a kinase inhibitor results in a more explicit difference between the kinase activity profiles of the different tissues.

### Example 2:

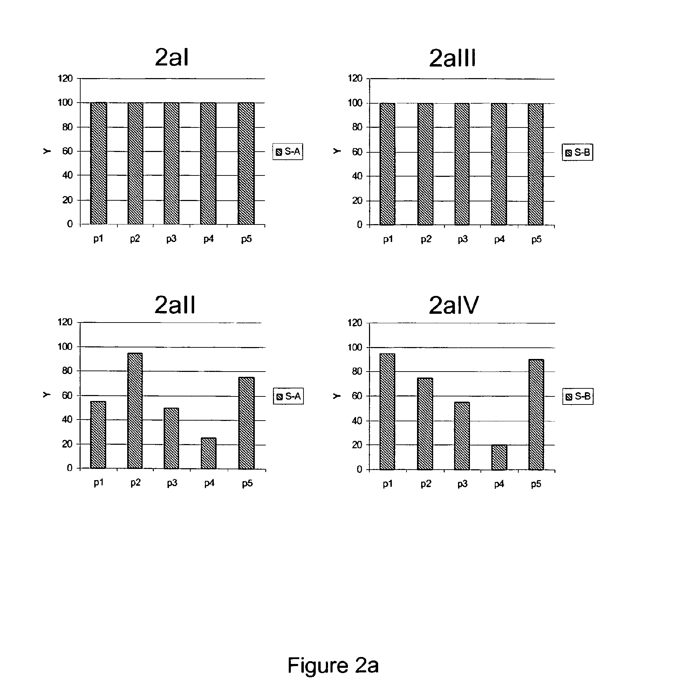

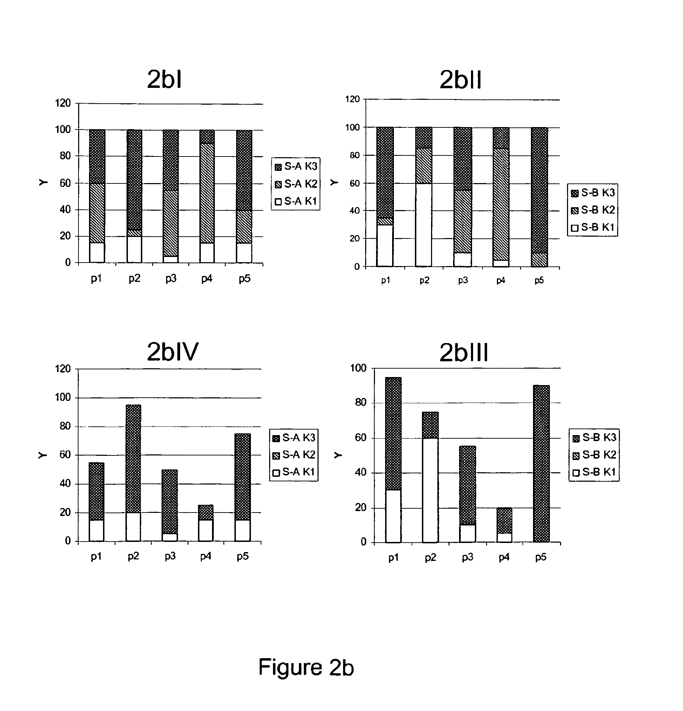

In order to generate an explanation for the unexpected outcome of the experiment described in example 1, a virtual experiment is performed. This mathematical experiment is to show and explain why phosphorylation inhibition profiles, are more discriminative in showing differences between tumor cells of different types (either derived from cell lines or patient derived tumor tissue) than basal (not treated with kinase inhibitor). In this experiment the complexity of a chip is reduced to only 5 peptides, for purpose of clarity. Figure 2 shows the results of an experiment when two samples A and B are profiled on such a 5-peptide peptide microarray for kinase activity. Figure 2aI and 2aII show identical profiles for sample A and B assuming that each peptide is maximally phosphorylated, however when the samples are analysed in the presence of an inhibitor discriminative profiles are obtained (2aIII and 2aIV, for sample A and B, respectively). This result is explained in the following way. One peptide is not per se phosphorylated by one single kinase. Kinases are promiscuous in recognizing and thus phosphorylating peptide substrates. However, kinases of different identity do have a preference for a certain peptide. As a consequence peptide 2, for example, is phophorylated by three kinases (kinase 1,2,3) in sample 1, but primarily by kinase 3, moderately by kinase 2 and only slightly by kinase 3. The ratios of contribution of each kinase to the phosphorylation of a peptide are dependent on their activities. In sample 2 the ratio of these activities can be different. This situation is exemplified by the graphs in figure 2b1 and 2b2, here for all 5 peptides. When a sample is profiles in the presence of a kinase inhibitor the ratio of activities of the kinases will change, as the inhibitor will decrease the activity of one kinase more than of the other kinase. This will, consequently, be reflected in the overall peptide phosphorylation levels. Additionally, the effect of the presence of inhibitor will be different in sample A from sample B, if the ratio's of kinase activities are different as well. As a result, if the ratio of activities of different kinases present in a sample are different from the kinase activities in an other sample, these differences will be more pronounced by peptide microarray analysis in presence of inhibitor, than by basal profiles (in absence of inhibitor).

### Example 3:

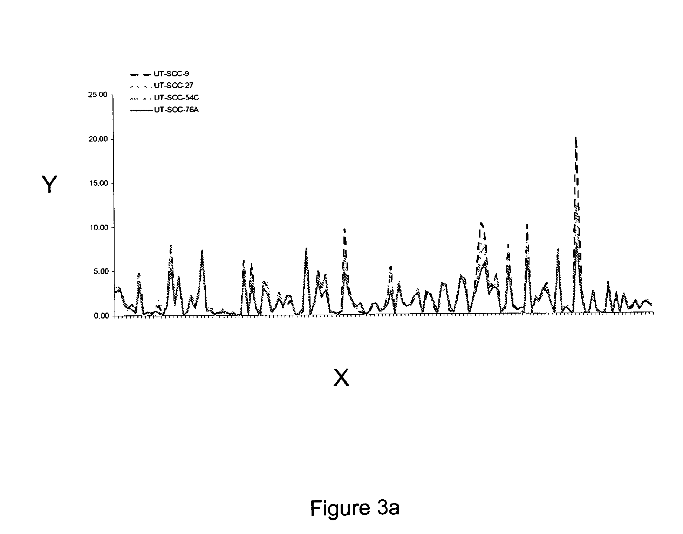

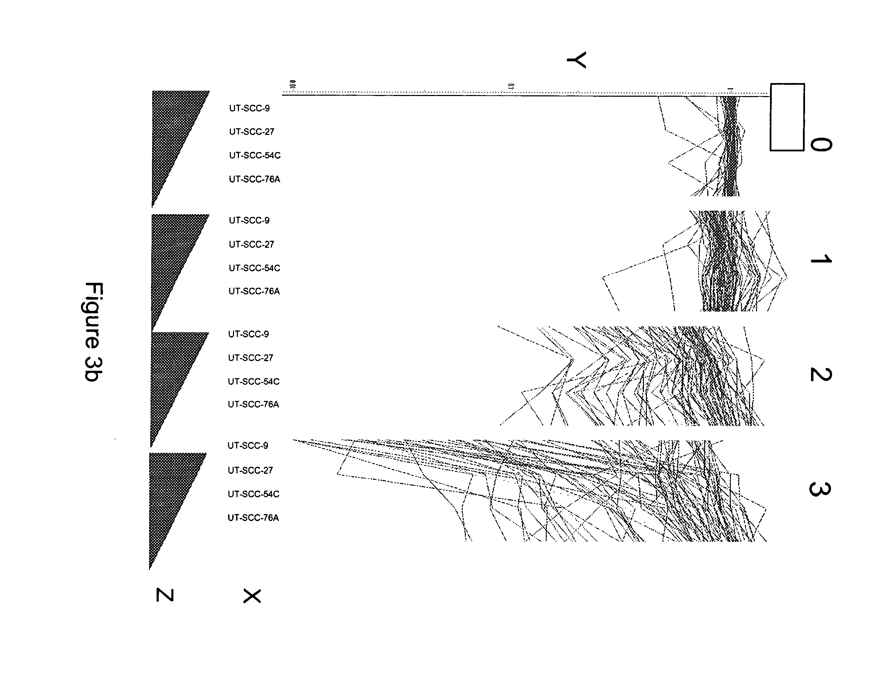

In order to assess the effect of applying inhibition profiles to discriminate different types of tumor cells, 4 cell lines were profiled in the absence of kinase inhibitor and in the presence of Iressa or PP1, kinase inhibitors that are known to inhibit EGFR, Src family kinases, respectively, or Staurosporin, an inhibitor which is known to inhibit multiple kinase activities. In these kinase activity profiling and inhibition experiments the same method was used as described above in example 1. The 4 cell lines chosen, UT-SCC-9, UT-SCC-27, UT-SCC-54C, and UT-SCC-76 are patient derived cell lines, known to respond differently to radiation (in a model for therapy in head and neck cancer, squamous cell carcinomas, especially). These lines are responsive (UT-SCC-9), moderate responsive (UT-SCC-27) or non-responsive (resistant; UT-SCC-54C and UT-SCC-76) to radiation. **Figure 3a** shows the phosphorylation profiles of the different peptides on the pamchip (on the x-axis, X) expressed (Y) in normalized fluorescence intensity (in arbitrary units) obtained in the absence of any kinase inhibitor. These profiles are very similar. However, when inhibition profiles are generated, as is shown in **figure 3b** in the presence of Iressa (1), PP1 (2), or Staurosporin (3) pronounced differences between the cells lines (X) were found, especially when PP1 (2) or Staurosporin (3) was used. The data shown are from a selected set of peptides of which the ratios of phosphorylation in the absence (0) and presence of the inhibitor are represented. This clearly shows that the presence of a kinase inhibitor results in a more explicit difference between the kinase activity profiles of the different tissues.

Surprisingly, the degree of inhibition (ratio<1) appeared to correlate with the known responsiveness to radiation (represented by Z). As a result the peptides identified are response markers, which can be used to predicting the response of the tumor cells to therapy, here being radiation. But the same approach could be used to identify peptide markers of which the degree of inhibition predicts a tumor cell line or patient's tumor response to, for example, kinase inhibitor therapy.

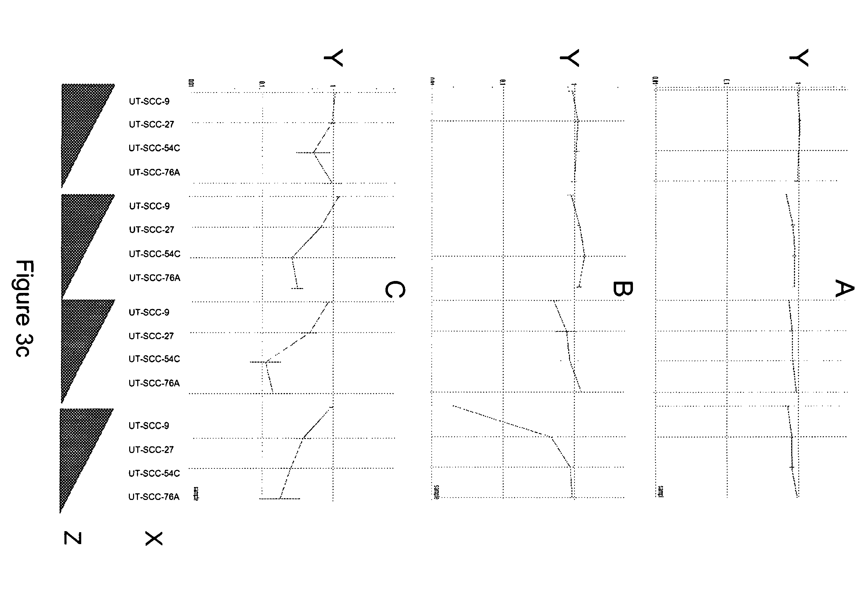

**Figure 3c** shows the same experiment as in figure 3b, and expressed in the same way, but only single peptide effects are shown for peptide VEGFR2_Y951 (A), JAK1_Y1022_Y1023 (B), and DDR1_Y513 (C), respectively. As these peptides show different responses on phosphorylation to inhibitor treatment, this shows that the effects are peptide specific, and therefore kinase specific. Furthermore, figure 3b and 3c show that the correlation between peptide phosphorylation inhibition (Y) and radiation response (Z) can be direct (3b) or a reverse correlation (3c). These peptides JAK1_Y1022_Y1023 and DDR1_Y51 are representatives of different classes of response markers.

### Example 4:

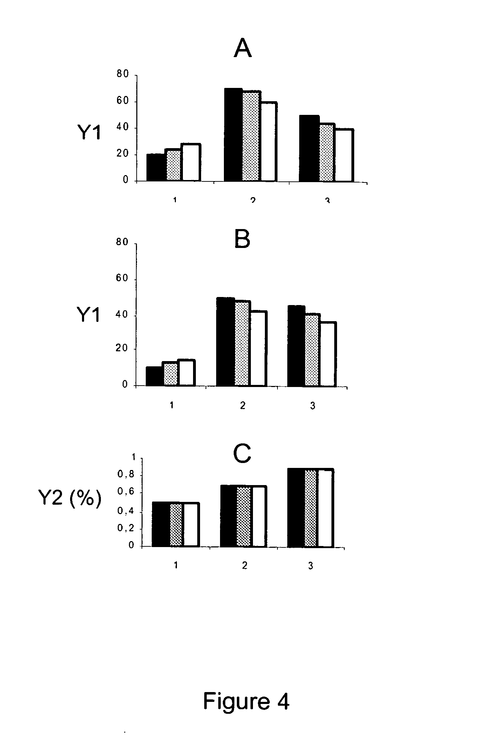

An additional advantage of inhibition experiments is that besides their enhanced discriminatory power to differentiate samples with different and complex kinase activities, inhibition experiments have a reduced variance in replicates resulting in more robust measurements. In order to generate an explanation for this, a virtual experiment is performed. This mathematical experiment is to show the reduced variance, here expressed as CV values (coefficient of variance), when inhibition profiles are generated in comparison to basal phosphorylation profiles without kinase inhibitors. In this experiment the complexity of a chip is reduced to only 3 peptides, for purpose of clarity. Table 2 shows the results of an experiment, when one sample is profiled on such a 3-peptide array for kinase activity in the absence of a kinase inhibitor. The data of table 2 are represented in figure 4 (Y1 = peptide phosphorylation expressed as fluorescence signal, Y2= peptide phosphorylation inhibition expressed as ratio (%) of signal in absence of inhibitor and presence of inhibitor). From this example it is clear that the CV values are much lower (table 2) in case inhibitors are used, and results are expressed as percentage of inhibition, than when absolute values are used generated in absence of an inhibitor.

**Table 2**

| | peptide 1 | peptide 2 | peptide 3 |
|---|---|---|---|
| non-treated (not inhibited) phosphorylation values from a 3-peptide array (data expressed as absolute signal) | | | |
| measurement | | | |
| 1 | 20 | 70 | 50 |
| 2 | 25 | 68 | 45 |
| 3 | 28 | 60 | 40 |
| **CV(%)** | **17%** | **8%** | **11%** |
| | | | |

| kinase inhibitor treatment resulting in 50%, 30%, and 10% inhibition of peptide 1,2,3, respectively (data expressed as absolute signal) | | | |
|---|---|---|---|
| measurement | | | |
| 1 | 10 | 49 | 45 |
| 2 | 13 | 48 | 41 |
| 3 | 14 | 42 | 36 |
| **CV(%)** | **17%** | **8%** | **11%** |
| | | | |

| kinase inhibitor treatment resulting in 50%, 30%, and 10% inhibition of peptide 1,2,3, respectively data expressed as ratio (%) of inhibitor treated to non-treated | | | |
|---|---|---|---|
| measurement | | | |
| 1 | 0.5 | 0.7 | 0.9 |
| 2 | 0.5 | 0.7 | 0.9 |
| 3 | 0.5 | 0.7 | 0.9 |
| **CV(%)** | **0%** | **0%** | **0%** |

### Example 5:

The present example provides measurements of kinase activity of Non Small Cell Lung Cancer (NSCLC) tissue in the presence and in the absence of the protein kinase inhibitor gefitinib.

The inventors have found that the survival prognosis can be classified by testing inhibition of kinase activity by studying kinase phosphorylation activity and inhibition levels in resection tissue from the tumor. The tumor content for each of the samples was higher than 70 %, based on HE staining. 6 samples were from patients having a short survival (less than 9 months), 8 samples were from patients with a long survival period (over 95 months.

6 coupes of 10 µm thickness from tumor tissue were lysed in 100 microliter Mammalian Extration Buffer (M-PER) containing phosphatase and protease inhibitors. After 30 minutes of lysis on ice, and centrifugation for 15 min at 4 °C, the supernatants were aliquotted and frozen. For each sample. 10 µg of protein diluted in the lysis solution, was pipetted into a reaction mixture composed of 1x ABL buffer (10xAbl buffer (New England Biolabs, cat.nr B6050S -100 mM MgCl2, 10 mM EGTA, 20 mM DTT and 0.1 % Brij 35 in 500 mM Tris/HCl, pH 7.5), 0,1% Bovine Serum Albumin, 100 µM ATP, 7.5 µg/ml anti-phosphotyrosine antibody to an end volume of 40 microliter. Before incubation of the lysate reaction mixtures on the PamChip substrate array a blocking step was carried out on the substrate arrays with 2% bovine serum albumin. After washing 3x with Abl buffer and loading of the lysate reaction mixtures into substrate arrays comprising 140 protein protein kinase substrates, including the 19 protein kinase peptide substrates as listed in Table 1, incubation was commenced thereby measuring the kinase activity of the sample. Each tumor tissue lysates was tested in three technical replicates on the substrate arrays without inhibitor and in three technical replicates with 1 uM of inhibitor gefitinib. During 60 cycles of pumping the lysate reaction mixture through the array, peptide phosphorylation was detected by an antibody present in the lysate reaction mixture. Real time data were obtained by measuring fluorescence of the bound anti-phosphotyrosine antibody after each 5 cycles. Images of the array were taken during the incubation of the array and after 60 cycles of incubation. After 60 cycles of incubation and imaging, the antibody mixture was removed and the array was washed. Images were collected at different exposure times. Signals for each spot on the image were quantified. Image quantification and data processing was conducted with dedicated PamGene software (Evolve and Bionavigator).

The results of the incubation of the NSCLC samples on a PamChip® with and without the protein kinase inhibitor gefitinib at a concentration of 1 µmol/L show that the addition of gefitinib as protein kinase inhibitor provides a number of essential differences between the phosphorylation profiles with and without the protein kinase inhibitor, which enables the differentiation between the different survival prognoses of NSCLC patients (data not shown).

The present example shows that the kinase activity profiles in combination with a kinase inhibitor clearly provide a differentiated phosphorylation profile.

The present example shows that the kinase activity profiles in combination with a kinase inhibitor clearly provide a differentiated phosphorylation profile.

### Example 6:

The present example shows the survival prognosis of NSCLC patients.

Tumor samples obtained from 2 classes of patients: short term survivors (6 samples) and long term survivors (8 samples) were incubated as described in example 5 on PamChip® arrays. The fluorescence from the spots in the images was quantified for quantitative purposes.

For each peptide the ratio of inhibited versus not inhibited signal was calculated and a one way ANOVA was performed to identify peptides with a significant difference between the long and short term survivors. Table 3 enlists 19 out of the 140 peptides that have a probability (p) for equal means over the 2 patient classes of p < 0.01. A heatmap showing the ratio of inhibited versus not inhibited phosphorylation signal in each of the peptide markers listed in Table 3, is provided in Figure 6. Whereas the dotted squares indicate a positive ratio and the non-dotted squares a negative ratio, the grey intensity of the squares is proportional to the ratio level.

The present example shows that the kinase activity profiles in combination with a kinase inhibitor can be used to identify early stage NSCLC patients with poor prognosis. The fact that a distinction can be made between long and short term survivors, based on effect of inhibitor gefitinib in the patients proves that the use of the peptide set as listed in Table 3 enables to determine the prognosis of survival in NSCLC and according to the prognosis select patients that might benefit from specific adjuvant therapy.

**Table 3**

| **Seq. Id. No.** | **Name** | **Sequence** |
|---|---|---|
| 13 | CBL_693_705 | EGEEDTEYMTPSS |
| 78 | LTK_669_681 | RDIYRASYYRRGD |
| 19 | CREB1_122_134 | OKRREILSRRPSY |
| 137 | TEC_512_524 | RYFLDDQYTSSSG |
| 117 | RAF1_331_343 | RPRGQRDSSYYWE |
| 139 | TYRO3_679_691 | KIYSGDYYRQGCA |
| 89 | NCF1_313_325 | QRSRKRLSQDAYR |
| 80 | MBP_259_271 | FGYGGRASDYKSA |
| 49 | ERBB2_1241_1253 | PTAENPEYLGLDV |
| 50 | ERBB2_870_882 | LDIDETEYHADGG |
| 90 | NPT2A_501_513 | AKALGKRTAKYRW |
| 10 | C1R_199_211 | TEASGYISSLEYP |
| 91 | NTRK1_489_501 | HIIENPQYFSDAC |
| 94 | NTRK2_699_711 | RDVYSTDYYRVGG |
| 71 | JAK1_1015_1027 | AIETDKEYYTVKD |
| 152 | VGFR2_944_956 | RFRQGKDYVGAIP |
| 67 | INSR_1348_1360 | SLGFKRSYEEHIP |
| 109 | PGFRB_771_783 | YMAPYDNYVPSAP |
| 31 | EGFR_1103_1115 | GSVQNPVYHNQPL |

## Claims

1. A method for diagnosing, determining the prognosis and/or predicting the treatment outcome of a cancer pathology, said method comprising the steps of:
a) determining kinase activity of one or more clinical tissue lysate or cell lysate samples from a patient by incubating said samples with ATP on an array of two or more protein kinase substrates, thereby generating a kinase activity profile;
b) determining kinase activity of one or more clinical tissue lysate or cell lysate samples from a patient by incubating said samples with a kinase inhibitor and ATP on an array of two or more protein kinase substrates, thereby generating a kinase activity profile;
c) inferring the influence of said kinase inhibitor on the kinase activity profile, whereby an inhibition profile is generated by comparing the kinase activity profiles obtained in step a) and b); and,
d) comparing inhibition profiles obtained from clinical reference tissue lysate or cell lysate samples with the inhibition profile obtained in step c), thereby diagnosing, determining the prognosis and/or predicting the treatment outcome of a cancer pathology.

2. The method according to claim 1, further comprising the presence of a second protein kinase inhibitor in steps a) and b).

3. The method according to any of claims 1 or 2, wherein said array is a flow-through array.

4. The method according to any of claims 1 to 3, wherein said protein kinase substrates are at least two protein kinase substrates selected from the group consisting of the protein kinase substrates with any of Seq. Id. No. 1 to 157.

5. The method according to any of claims 1 to 4, wherein said protein kinase substrates consist of the peptide kinase substrates with any of Seq. Id. No. 152, 71, and 23.

6. The method according to any of claims 1 to 5 additionally comprising the step of comparing kinase activity profiles obtained from clinical reference tissue lysate or cell lysate samples with the kinase activity profiles of said clinical tissue lysate or cell lysate samples.

7. Method according to any of claims 1 to 6, for the prediction of the response of a patient to said kinase inhibitor.

8. Method according to any of claims 1 to 6, to determine the clinical outcome the prediction of clinical efficacy, side effects or adverse effects of a kinase inhibitor therapy of a cancer pathology.

9. Method according to any of the previous claims wherein in steps a) and b) the kinase activity of three or more different clinical tissue lysate or cell lysate samples are determined.

10. A computer readable medium comprising a computer program comprising instructions to carry out the method for diagnosing, determining the prognosis or predicting the treatment outcome of a cancer pathology by carrying out steps c) and d) according to any of claims 1 to 9, when loaded on a computer.

## Patentansprüche

1. Verfahren zur Diagnose, Bestimmung der Prognose und/oder Vorhersage des Behandlungsergebnisses des Krankheitsbilds einer Krebserkrankung, wobei man in den Verfahrensschritten:
a) Kinase-Aktivität einer oder mehrerer klinischer Gewebelysat- oder Zelllysatproben aus einem Patienten bestimmt, indem man die Proben mit ATP auf einem Array von zwei oder mehr Proteinkinase-Substraten inkubiert, wodurch ein Kinase-Aktivitätsprofil erzeugt wird;
b) Kinase-Aktivität einer oder mehrerer klinischer Gewebelysat- oder Zelllysatproben aus einem Patienten bestimmt, indem man die Proben mit einem Kinase-Inhibitor und ATP auf einem Array von zwei oder mehr Proteinkinase-Substraten inkubiert, wodurch ein Kinase-Aktivitätsprofil erzeugt wird;
c) den Einfluss des Kinase-Inhibitors auf das Kinase-Aktivitätsprofil ableitet, wobei ein Hemmprofil durch Vergleichen der in Schritt a) und b) erhaltenen Kinase-Aktivitätsprofile erzeugt wird; und
d) von klinischen Referenz-Gewebelysat- oder -Zelllysatproben erhaltene Hemmprofile mit dem in Schritt c) erhaltenen Hemmprofil vergleicht, wodurch das Krankheitsbild einer Krebserkrankung diagnostiziert, dessen Prognose bestimmt und/oder das Behandlungsergebnis davon vorhergesagt wird.

2. Verfahren nach Anspruch 1, ferner umfassend das Vorliegen eines zweiten Proteinkinase-Inhibitors in den Schritten a) und b).

3. Verfahren nach Anspruch 1 oder 2, wobei es sich bei dem Array um ein Durchfluss-Array handelt.

4. Verfahren nach einem der Ansprüche 1 bis 3, wobei es sich bei den Proteinkinase-Substraten um wenigstens zwei aus der aus den Proteinkinase-Substraten mit einer der Seq. Id. Nr. 1 bis 157 bestehenden Gruppe aus-gewählte Proteinkinase-Substrate handelt.

5. Verfahren nach einem der Ansprüche 1 bis 4, wobei die Proteinkinase-Substrate aus den Peptidkinase-Substraten mit einer der Seq. Id. Nr. 152, 71 und 23 bestehen.

6. Verfahren nach einem der Ansprüche 1 bis 5, bei dem man in einem zusätzlichen Schritt von klinischen Referenz-Gewebelysat- oder -Zelllysatproben erhaltene Kinase-Aktivitätsprofile mit den Kinase-Aktivitätsprofilen der klinischen Gewebelysat- oder Zelllysatproben vergleicht.

7. Verfahren nach einem der Ansprüche 1 bis 6, zur Vorhersage des Ansprechens eines Patienten auf den Kinase-Inhibitor.

8. Verfahren nach einem der Ansprüche 1 bis 6, zur Bestimmung des klinischen Ergebnisses, der Vorhersage klinischer Wirksamkeit, von Nebenwirkungen oder negativen Effekten einer Kinase-Inhibitor-Therapie des Krankheitsbilds einer Krebserkrankung.

9. Verfahren nach einem der vorhergehenden Ansprüche, wobei in den Schritten a) und b) die Kinase-Aktivität von drei oder mehr unterschiedlichen klinischen Gewebelysat- oder Zelllysatproben bestimmt werden.

10. Computer-lesbarer Datenträger, umfassend ein Computerprogramm, umfassend Anweisungen zur Durchführung des Verfahrens zur Diagnose, Bestimmung der Prognose oder Vorhersage des Behandlungsergebnisses des Krankheitsbilds einer Krebserkrankung, indem nach Laden auf einem Computer die Schritte c) und d) nach einem der Ansprüche 1 bis 9 durchgeführt werden.

## Revendications

1. Procédé pour diagnostiquer, déterminer le pronostic et/ou prédire le résultat de traitement d'une pathologie cancéreuse, ledit procédé comprenant les étapes de :
a) détermination de l'activité kinase d'un ou plusieurs échantillons cliniques de lysat de tissu ou lysat de cellules d'un patient par incubation desdits échantillons avec de l'ATP sur une puce de deux substrats de protéine kinase ou plus, de manière à générer un profil d'activité kinase ;
b) détermination de l'activité kinase d'un ou plusieurs échantillons cliniques de lysat de tissu ou lysat de cellules d'un patient par incubation desdits échantillons avec un inhibiteur de kinase et de l'ATP sur une puce de deux substrats de protéine kinase ou plus, de manière à générer un profil d'activité kinase ;
c) déduction de l'influence dudit inhibiteur de kinase sur le profil d'activité kinase, un profil d'inhibition étant généré par comparaison des profils d'activité kinase dans l'étape a) et b) ; et,
d) comparaison des profils d'inhibition obtenus à partir d'échantillons cliniques de référence de lysat de tissu ou de lysat de cellules au profil d'inhibition obtenu dans l'étape c), de manière à diagnostiquer, déterminer le pronostic et/ou prédire le résultat de traitement d'une pathologie cancéreuse.

2. Procédé selon la revendication 1, comprenant en outre la présence d'un deuxième inhibiteur de protéine kinase dans les étapes a) et b).

3. Procédé selon l'une quelconque des revendications 1 ou 2, dans lequel ladite puce est une puce à circulation.

4. Procédé selon l'une quelconque des revendications précédentes 1 à 3, dans lequel lesdits substrats de protéine kinase sont au moins deux substrats de protéine kinase choisis dans le groupe constitué des substrats de protéine kinase ayant l'un quelconque de Seq. Id. N° 1 à 157.

5. Procédé selon l'une quelconque des revendications 1 à 4, dans lequel lesdits substrats de protéine kinase sont constitués des substrats de peptide kinase ayant l'un quelconque de Seq. Id. N° 152, 71, et 23.

6. Procédé selon l'une quelconque des revendications 1 à 5, comprenant en outre l'étape de comparaison des profils d'activité kinase obtenus à partir des échantillons cliniques de référence de lysat de tissu ou de lysat de cellules aux profils d'activité kinase desdits échantillons de lysat de tissu ou de lysat de cellules.

7. Procédé selon l'une quelconque des revendications 1 à 6, pour la prédiction de la réponse d'un patient audit inhibiteur de kinase.

8. Procédé selon l'une quelconque des revendications 1 à 6, pour déterminer le résultat clinique, la prédiction de l'efficacité clinique, les effets secondaires ou les effets indésirables d'un traitement avec un inhibiteur de kinase d'une pathologie cancéreuse.

9. Procédé selon l'une quelconque des revendications précédentes dans lequel, dans les étapes a) et b), l'activité kinase de trois échantillons cliniques de lysat de tissu ou de lysat de cellules ou plus est déterminée.

10. Support lisible par ordinateur comprenant un programme informatique comprenant des instructions pour conduire le procédé pour diagnostiquer, déterminer le pronostic ou prédire le résultat du traitement d'une pathologie cancéreuse en conduisant les étapes c) et d) selon l'une quelconque des revendications 1 à 9, lorsqu'il est chargé sur un ordinateur.
